# EUROPÄISCHE PATENTSCHRIFT

(11) **EP 2 078 095 B1**
(45) Veröffentlichungstag und Bekanntmachung des Hinweises auf die Patenterteilung: **03.04.2013**
(21) Anmeldenummer: 07800165.8
(22) Anmeldetag: 04.09.2007
(51) Int. Cl.: C12Q 1/68

(54) **BAKTERIENNACHWEIS**
DETECTION OF BACTERIA
DÉTECTION DE BACTÉRIES

(30) Priorität: 07.09.2006 AT 14962006
(43) Veröffentlichungstag der Anmeldung: 15.07.2009
(73) Patentinhaber: Österreichisches Rotes Kreuz Landesverband Oberösterreich, 4020 Linz (AT)
(72) Erfinder: DANZER, Martin, A-4813 Altmünster (AT); POLIN, Helene, A-4800 Attnang-Puchheim (AT); HOFER, Katja, A-4100 Ottensheim (AT); FIEDLER, Brigitte, A-4052 Ansfelden (AT); RADLER, Juliane, A-4172 St. Johann/Wbg. (AT); ROSENHAMMER, Katrin, A-4020 Linz (AT); ATZMÜLLER, Sabine, A-4173 St. Veit im Mühlkreis (AT); GABRIEL, Christian, A-4203 Altenberg (AT)
(74) Vertreter: Secklehner, Günter
(86) Internationale Anmeldenummer: PCT/AT2007/000420
(87) Internationale Veröffentlichungsnummer: WO 2008/028210

(56) Entgegenhaltungen:
- EP-A- 1 473 370
- WO-A-02/10444
- WO-A-02/070728
- WO-A-2004/046375
- US-A1- 2005 037 408

### Sequenzprotokoll

<110> Österreichisches Rotes Kreuz Landesverband Oberösterreich
   Körnerstrasse 28
   4020 Linz
<120> Bakteriennachweis
<160> 35
<210> 1
   <211> 19
   <212> DNA
   <213> künstliche Sequenz
<220>
   <223> Beschreibung der künstlichen Sequenz: Primer
<400> 1
   tcctacggga ggcagcagt 19
<210> 2
   <211> 26
   <212> DNA
   <213> künstliche Sequenz
<220>
   <223> Beschreibung der künstlichen Sequenz: Primer
<400> 2
   ggactaccag ggtatctaat cctgtt 26
<210> 3
   <211> 18
   <212> DNA
   <213> künstliche Sequenz
<220>
   <223> Beschreibung der künstlichen Sequenz: Primer
<400> 3
   gcctgggttc cctgttcc 18
<210> 4
   <211> 19
   <212> DNA
   <213> künstliche Sequenz
<220>
   <223> Beschreibung der künstlichen Sequenz: Primer
<400> 4
   cgacgtaccc ctgacatgg 19
<210> 5
   <211> 22
   <212> DNA
   <213> künstliche Sequenz
<220>
   <223> Beschreibung der künstlichen Sequenz: Oligonukleotid
<400> 5
   gtattaccgc ggctgctggc ac 22
<210> 6
   <211> 28
   <212> DNA
   <213> künstliche Sequenz
<220>
   <223> Beschreibung der künstlichen Sequenz: Oligonukleotid
<400> 6
   tagttagccg tggctttctg gttagata 28
<210> 7
   <211> 28
   <212> DNA
   <213> künstliche Sequenz
<220>
   <223> Beschreibung der künstlichen Sequenz: Oligonukleotid
<400> 7
   tagttagccg tccctttctg gttagata 28
<210> 8
   <211> 28
   <212> DNA
   <213> künstliche Sequenz
<220>
   <223> Beschreibung der künstlichen Sequenz: Oligonukleotid
<400> 8
   tagttagccg tggctttctg gttaggta 28
<210> 9
   <211> 22
   <212> DNA
   <213> künstliche Sequenz
<220>
   <223> Beschreibung der künstlichen Sequenz: Oligonukleotid
<400> 9
   cggtgcttct tctgcgagta ac 22
<210> 10
   <211> 24
   <212> DNA
   <213> künstliche Sequenz
<220>
   <223> Beschreibung der künstlichen Sequenz: Oligonukleotid
<400> 10
   cggtgcttat tctttaggta ccgt 24
<210> 11
   <211> 24
   <212> DNA
   <213> künstliche Sequenz
<220>
   <223> Beschreibung der künstlichen Sequenz: Oligonukleotid
<400> 11
   cggtgcttat tctgttggta acgt 24
<210> 12
   <211> 25
   <212> DNA
   <213> künstliche Sequenz
<220>
   <223> Beschreibung der künstlichen Sequenz: Oligonukleotid
<400> 12
   caggccaatg tctcaccaag ctctg 25
<210> 13
   <211> 25
   <212> DNA
   <213> künstliche Sequenz
<220>
   <223> Beschreibung der künstlichen Sequenz: Oligonukleotid
<400> 13
   acggtagccg ggttatgaag cgccg 25
<210> 14
   <211> 23
   <212> DNA
   <213> künstliche Sequenz
<220>
   <223> Beschreibung der künstlichen Sequenz: Oligonukleotid
<400> 14
   agggtagtgg gtaagaagcg ccg 23
<210> 15
   <211> 23
   <212> DNA
   <213> künstliche Sequenz
<220>
   <223> Beschreibung der künstlichen Sequenz: Oligonukleotid
<400> 15
   tgaataagcg ccggctaagc gcg 23
<210> 16
   <211> 21
   <212> DNA
   <213> künstliche Sequenz
<220>
   <223> Beschreibung der künstlichen Sequenz: Oligonukleotid
<400> 16
   atgtaccgta tgaataagga t 21
<210> 17
   <211> 21
   <212> DNA
   <213> künstliche Sequenz
<220>
   <223> Beschreibung der künstlichen Sequenz: Oligonukleotid
<400> 17
   atgtataata tgaataagga t 21
<210> 18
   <211> 21
   <212> DNA
   <213> künstliche Sequenz
<220>
   <223> Beschreibung der künstlichen Sequenz: Oligonukleotid
<400> 18
   atgtaccttg tgaataagca t 21
<210> 19
   <211>21
   <212> DNA
   <213> künstliche Sequenz
<220>
   <223> Beschreibung der künstlichen Sequenz: Oligonukleotid
<400> 19
   atgtacccta tgaataagca t 21
<210> 20
   <211> 21
   <212> DNA
   <213> künstliche Sequenz
<220>
   <223> Beschreibung der künstlichen Sequenz: Oligonukleotid
<400> 20
   aagtatcgta cgaataagga t 21
<210> 21
   <211> 24
   <212> DNA
   <213> künstliche Sequenz
<220>
   <223> Beschreibung der künstlichen Sequenz: Oligonukleotid
<400> 21
   agcgtaccag gtaaagaagc accg 24
<210> 22
   <211> 24
   <212> DNA
   <213> künstliche Sequenz
<220>
   <223> Beschreibung der künstlichen Sequenz: Oligonukleotid
<400> 22
   agcgtaccgg gtaaagaagc accg 24
<210> 23
   <211> 28
   <212> DNA
   <213> künstliche Sequenz
<220>
   <223> Beschreibung der künstlichen Sequenz: Oligonukleotid
<400> 23
   tacccttgga ggaagccgcg gctaacta 28
<210> 24
   <211> 26
   <212> DNA
   <213> künstliche Sequenz
<220>
   <223> Beschreibung der künstlichen Sequenz: Oligonukleotid
<400> 24
   tacctaccta aagcaccggc taacta 26
<210> 25
   <211> 26
   <212> DNA
   <213> künstliche Sequenz
<220>
   <223> Beschreibung der künstlichen Sequenz: Oligonukleotid
<400> 25
   tacctgccta aagcaccggc taacta 26
<210> 26
   <211> 22
   <212> DNA
   <213> künstliche Sequenz
<220>
   <223> Beschreibung der künstlichen Sequenz: Oligonukleotid
<400> 26
   ggtagcgggg gaagaagcgc cg 22
<210> 27
   <211> 24
   <212> DNA
   <213> künstliche Sequenz
<220>
   <223> Beschreibung der künstlichen Sequenz: Oligonukleotid
<400> 27
   ctgtaccatt ttgaataagt gacg 24
<210> 28
   <211> 28
   <212> DNA
   <213> künstliche Sequenz
<220>
   <223> Beschreibung der künstlichen Sequenz: Oligonukleotid
<400> 28
   taccctgtga ggaagccccg gctaacta 28
<210> 29
   <211> 28
   <212> DNA
   <213> künstliche Sequenz
<220>
   <223> Beschreibung der künstlichen Sequenz: Oligonukleotid
<400> 29
   taccatagga ggaagccccg gctaaata 28
<210> 30
   <211> 24
   <212> DNA
   <213> künstliche Sequenz
<220>
   <223> Beschreibung der künstlichen Sequenz: Oligonukleotid
<400> 30
   catgtactct acgaataagt atcg 24
<210> 31
   <211> 24
   <212> DNA
   <213> künstliche Sequenz
<220>
   <223> Beschreibung der künstlichen Sequenz: Oligonukleotid
<400> 31
   acggtaggtg cagagaaagc gccg 24
<210> 32
   <211> 27
   <212> DNA
   <213> künstliche Sequenz
<220>
   <223> Beschreibung der künstlichen Sequenz: Oligonukleotid
<400> 32
   ctgaatgtac tggaagaata aggatcg 27
<210> 33
   <211> 26
   <212> DNA
   <213> künstliche Sequenz
<220>
   <223> Beschreibung der künstlichen Sequenz: Oligonukleotid
<400> 33
   ctgaatgtac ccaagaataa ggatcg 26
<210> 34
   <211> 25
   <212> DNA
   <213> künstliche Sequenz
<220>
   <223> Beschreibung der künstlichen Sequenz: Oligonukleotid
<400> 34
   acggtagtcg tgcgaataag ccccg 25
<210> 35
   <211> 24
   <212> DNA
   <213> künstliche Sequenz
<220>
   <223> Beschreibung der künstlichen Sequenz: Oligonukleotid
<400> 35
   actgtaccat ttgaataagt atcg 24

## Beschreibung

Die vorliegende Erfindung beschreibt Oligonukleotidsequenzen zum Nachweis von Bakterienkontaminationen von einer Länge von 10 bis 30 Basen, ein Verfahren zur Detektion zumindest einer Bakterienkontamination sowie einen Kit zum Nachweis von Bakterienkontaminationen jeweils vorzugsweise in einer physiologischen Probe.

Die Erfindung betrifft das Gebiet der Molekularbiologie und deren Anwendung in der klinischen Diagnostik. Genauer betrifft die Erfindung ein Verfahren und Reagenzien zur Amplifikation und zum Nachweis von Nukleinsäuren von Bakterien. Die Erfindung findet deshalb Anwendung beim Nachweis von Bakterien, Bakteriengruppen und Bakterienarten, generell auf dem Gebiet der klinischen Diagnostik und dem Gebiet der Molekularbiologie.

Im Bereich der Medizin, insbesondere im klinischen Bereich, stellen Bakterienkontaminationen eine große Bedrohung für die Patienten dar. Insbesondere muss bei der Bereitstellung von Blutkonserven und den daraus gewonnenen Bestandteilen, wie beispielsweise Blutplasma zur Herstellung diverser Arzneimittel oder Thrombozytenkonzentraten, streng darauf geachtet werden Bakterienkontaminationen mit größtmöglicher Sicherheit ausschließen zu können. Solche Bakterienkontaminationen können beispielsweise schwere septische Reaktionen bei Patienten verursachen.

Die PCR ist ein Verfahren zur Amplifikation spezifischer Nukleinsäuresequenzen und ermöglicht den raschen Nachweis von Nukleinsäuren, die in einer Probe vorhanden sind, welche zuvor in einer nicht nachweisbar geringen Menge vorhanden waren.

Die Detektion von Nukleinsäuremolekülen in einer Probe wird in den verschiedensten Bereichen benötigt, beispielsweise in der Medizin, Qualitätskontrolle, Forschung, etc. Dabei kann es auch oft notwendig sein mehrere voneinander verschiedene Nukleinsäuremoleküle in einer einzigen Probe zu detektieren. Hierbei ist es aus Zeit- und Kostengründen wünschenswert die verschiedenen Nukleinsäuremoleküle gleichzeitig in einer Probe zu detektieren.

Allerdings ist die hochsensitive, molekularbiologische Detektion von Bakterien mittels Polymerasekettenreaktion (PCR) im Blut, häufig aufgrund der geringen Zellzahl im Verhältnis zum Probenvolumen beeinträchtigt. Zudem können falsch-positive Ergebnisse durch Amplifikation unspezifischer Sequenzen des humanen DNA-Hintergrunds auftreten. Die PCR ist ein Verfahren zur Amplifikation spezifischer Nukleinsäuresequenzen und ermöglicht den raschen Nachweis von Nukleinsäuren, die in einer Probe vorhanden sind, die zuvor in einer nicht nachweisbar geringen Menge vorhanden waren.

Die Detektion von Nukleinsäuremolekülen in einer Probe wird in den verschiedensten Bereichen benötigt, beispielsweise in der Medizin, Qualitätskontrolle, Forschung, etc. Dabei kann es auch oft notwendig sein mehrere voneinander verschiedene Nukleinsäuremoleküle in einer einzigen Probe zu detektieren. Hierbei ist es aus Zeit- und Kostengründen wünschenswert die verschiedenen Nukleinsäuremoleküle gleichzeitig in einer Probe zu detektieren.

So wird beispielsweise in der EP 1 366 195 B1 ein Verfahren zur simultanen Detektion von zumindest zwei voneinander verschiedenen Nukleinsäuremolekülen in einer Probe beschrieben, wobei in einem ersten Schritt eine Multiplex-PCR und in einem zweiten Schritt eine Hybridisierungsreaktion mit auf einem Microarray immobilisierten Sonden durchgeführt wird, wonach die hybridisierten PCR-Produkte detektiert und gegebenenfalls quantifiziert werden, sowie einen Microarray und einen Kit zur simultanen Detektion von zumindest zwei voneinander verschiedenen Nukleinsäuremolekülen in einer Probe. Die für die Hybridisierungsreaktion eingesetzten Sonden, die jeweils spezifisch von den voneinander verschiedenen amplifizierten Sequenzen der Nukleinsäuremoleküle hybridisieren, weisen Schmelztemperaturen auf, die voneinander um maximal 2 °C, vorzugsweise maximal 1 °C abweichen. Dadurch, dass sich die Schmelztemperaturen der für die Hybridisierungsreaktion eingesetzten Sonden um maximal 2 °C voneinander unterscheiden, ist es möglich, eine große Anzahl von Nukleinsäuremolekülen in einer Probe simultan zu detektieren, da für die Hybridisierungsreaktion für alle Sonden die selben Bedingungen bezüglich Temperatur aber auch Salzkonzentration, pH-Wert, etc. eingestellt werden.

Weiters beschreibt z.B. die US 6,664,081 B2 eine Methode, Oligonukleotide und einen Kit zur Detektion von Mycobakterienarten unter Verwendung der erfindungsgemäßen Oligonukleotide zur in-vitro Amplifikation der 16S rRNA-Sequenzen für viele Arten der Gattung Mycobakterium.

Die W02002/10444 beschreibt ein Real-time PCR Verfahren zum Nachweis von Mikroorganismen, wobei die SEQ ID NO: 1 und 2 als Primern verwendet wird und die SEQ ID NO: 6 als fluoreszenzmarkierte Probe.

Aufgabe der vorliegenden Erfindung ist ein Verfahren und Reagenzien zur Verfügung zu stellen, die einen sensitiven und spezifischen Nachweis von Bakterienkontaminationen, vorzugsweise in physiologischen Proben, ermöglichen.

Die Aufgabe wird jeweils eigenständig durch Oligonukleotide mit einer Nukleotidsequenz komplementär zu einer Nukleotidsequenz kodierend für eine Sequenz der hochkonservierten 16S rDNA Region eines Bakteriengenoms, wobei das Oligonukleotid mindestens 1 aufeinander folgende Basen einer Sequenz ausgewählt aus einer Gruppe umfassend. SEQ ID NO:5 bis SEQ ID NO:35 aufweist, einem Verfahren umfassend die Schritte i) Extraktion der Nukleinsäure, insbesondere Bakterien-DNA, ii) Amplifikation mit Primern und Detektion mit Oligonukleotiden, insbesondere fluoreszenzmarkierten Oligonukleotiden als Hybridisierungssonden, gemäß einem der Ansprüche 1 bis 26 in einer Realtime-PCR und iii) Auswertung über Schmelzkurvenanalyse und einem Kit enthaltend wenigstens ein Primerpaar zur Amplifikation, vorzugsweise gemäß Anspruch 32 und/oder 35, und zumindest ein Oligonukleotid gemäß einem der Ansprüche 1 bis 26, gegebenenfalls eine thermostabile DNA-Polymerase und dNTP-Lösungen, gelöst. Die Auswahl der erfindungsgemäßen bakterienspezifischen Oligonukleotide als Hybridisierungssonden gewährleistet die Detektion von insgesamt ca. 230 pathogenen Bakterien. Die Sensitivität des Verfahrens wird durch die Auswahl von spezifischen Primern für die Amplifikation von Nukleinsäuresequenzen in der hochkonservierten 16S rDNA-Region und die Spezifität durch die erfindungsgemäßen Oligonukleotide gewährleistet.

Dabei ist es besonders vorteilhaft eine 5'- und/oder 3'-Modifizierung, insbesondere mit Fluoreszenzmarkierung, durchzuführen um eine einfache Detektion, durch welche die Zugabe weiterer Reagenzien und somit ein abermaliges Öffnen des Reaktionsgefäßes vermieden werden kann, zu ermöglichen.

Vorzugsweise werden durch Oligonukleotide ausgewählt aus einer Gruppe umfassend SEQ ID NO:5, SEQ ID NO:6 bis SEQ ID NO:8, SEQ ID NO:13 bis SEQ ID NO:15, SEQ ID NO:28, SEQ ID NO:29 und SEQ ID NO:31 gram-positive Bakterienarten und durch Oligonukleotide ausgewählt aus einer Gruppe umfassend SEQ ID NO:5, SEQ ID NO:09 bis SEQ ID NO:11, SEQ ID NO:16 bis SEQ ID NO:27, SEQ ID NO:30 und SEQ ID NO:32 bis SEQ ID NO:35 gram-negative Bakterienarten nachgewiesen. Dadurch kann vorab eine Klassifizierung in gram-positive oder gram-negative Bakterienarten erfolgen.

Dabei ist es besonderes günstig, dass Oligonukleotid SEQ ID NO:6 spezifisch für die Gattung Enterococcus, Kurthia, Lactobacillus und/oder Listeria ist und selektiv Enterococcus avium, Enterococcus casseliflavus, Enterococcus durans, Enterococcus faecalis, Enterococcus faecium, Enterococcus gallinarum, Enterococcus hirae, Kurthia gibsonii, Kurthia sibirica, Lactobacillus acidophilus, Lactobacillus casei, Lactobacillus fermentum, Lactobacillus plantarum, Lactobacillus salivarius, Listeria monocytogenes und/oder Listeria pyogenes, vorzugsweise in einer physiologischen Probe, detektiert.

Vorteilhaft ist zudem auch, dass Oligonukleotid SEQ ID NO:7 spezifisch für die Gattung Leuconostoc und/oder Streptococcus ist und selektiv Leuconostoc citreum, Leuconostoc lactis, Streptococcus agalactiae, Streptococcus anginosus, Streptococcus bovis, Streptococcus mitis, Streptococcus pneumoniae, Streptococcus pyogenes, Streptococcus salivarius, Streptococcus sanguinis und/oder Streptococcus sp., vorzugsweise in einer physiologischen Probe, detektiert.

Oligonukleotid SEQ ID NO:8 ist vorteilhafterweise spezifisch für die Gattung Bacillus, Clostridium, Mycoplasma und/oder Staphylococcus und detektiert selektiv Bacillus anthracis, Bacillus circulans, Bacillus pumilus, Bacillus sphaericus, Bacillus subtilis, Bacteroides capillosus, Brevibacillus laterosporus, Clostridium bifermentans, Clostridium botulinum, Clostridium butyricum, Clostridium clostridioforme, Clostridium difficile, Clostridium novyi, Clostridium perfringens, Clostridium septicum, Clostridium sporogenes, Clostridium tetani, Erysipelothrix rhusiopathiae, Fusobacterium alocis, Gemella haemolysans, Mycoplasma orale, Mycoplasma pulmonis, Mycoplasma buccale, Staphylococcus aureus, Staphylococcus capitis, Staphylococcus cohnii, Staphylococcus epidermidis, Staphylococcus haemolyticus, Staphylococcus hominis, Staphylococcus lugdunensis, Staphylococcus xylosus und/oder Veillonella parvula, vorzugsweise in einer physiologischen Probe.

Vorzugsweise ist Oligonukleotid SEQ ID NO:9 spezifisch für die Gattung Acinetobacter, Actinomyces, Aeromonas, Anaerobiospirillum, Bartonella, Brucella, Citrobacter, Enterobacter, Haemophilus, Klebsiella, Kluyvera, Legionella, Pasteurella, Proteus, Rickettsia, Salmonella, Serratia, Shigella, Vibrio, Yersinia, und detektiert selektiv Acinetobacter baumannii, Acinetobacter calcoaceticus, Acinetobacter haemolyticus, Acinetobacter johnsonii, Acinetobacter junii, Acinetobacter lwoffii, Actinomyces meyeri, Actinomyces pyogenes, Aeromonas caciae, Aeromonas hydrophila, Aeromonas schubertii, Aeromonas veronii, Agrobacterium radiobacter, Alcaligenes faecalis, Anaerobiospirillum succiniciproducens, Anaerobiospirillum thomasii, Acranobacterium pyogenes, Bartonella bacilliformis, Bartonella henselae, Brucella abortus, Brucella melitensis, Calymmatobacterium granulomatis, Citrobacter amalonaticus, Citrobacter freundii, Coxiella burnetti, Edwardsiella tarda, Enterobacter aerogenes, Enterobacter cloacae, Enterobacter sakazakii, Enterobacter sp., Escherichia coli, Haemophilus aegypticus, Haemophilus aphrophilus, Haemophilus ducreyi, Haemophilus parahaemolyticus, Haemophilus parainfluenzae, Haemophilus paraphrophilus, Haemophilus segnis, Hafnia alvei, Klebsiella oxytoca, Klebsiella pneumoniae, Klebsiella rhinoscleromatis, Kluyvera ascorbata, Kluyvera cryocrescens, Legionella dumoffii, Legionella micdadei, Morganella morganii, Ochrobactrum anthropi, Pantoea agglomerans, Pasteurella gallinarum, Pasteurella pneumotropica, Plesiomonas shigelloides, Propionibacterium acnes, Proteus mirabilis, Proteus penneri, Proteus vulgaris, Pseudomonas putida, Rickettsia akari, Rickettsia australis, Rickettsia conorii, Salmonella choleraesius, Salmonella enterica, Salmonella paratyphi A, Salmonella paratyphi B, paratyphi C, Salmonella typhi, Salmonella typhinurium, Serratia ficaria, Serratia fonticola, Serratia grimesii, Serratia liquefaciens, Serratia marcescens, Serratia rudidaea, Shigella boydii, Shigella dysenteriae, Shigella flexneri, Shigella sonnei, Vibrio alginolyticus, Vibrio hollisae, Wigglesworthia glossinidia, Xanthomonas campestris, Yersinia enterocolitica, Yersinia pestis und/oder Yersinia pseudotuberculosis vorzugsweise in einer physiologischen Probe.

Dabei ist es günstig, dass Oligonukleotid SEQ ID NO:10 spezifisch für die Gattung Achromobacter, Actinomadura, Actinomyces, Afipia, Bordetella, Burkholderia, Campylobacter, Capnocytophaga, Comamonas, Corynebacterium, Ehrlichia, Fusobacterium, Methylobacterium, Mycobacterium, Neisseria, Nocardia, Oligella, Prevotella und/oder Rhodococcus ist und selektiv Achromobacter piechaudii, Achromobacter xylosoxidans, Actinomadura madurae, Actinomadura pelletieri, Actinomyces bovis, Actinomyces naeslundii, Actinomyces viscosus, Afipia broomeae, Afipia felis, Bacteroides gracilis, Bilophila wadsworthia, Bordetella bronchiseptica, Bordetella parapertussis, Bordetella pertussis, Burkholderia cepacia, Burkholderia gladioli, Burkholderia mallei, Burkholderia pseudomallei, Campylobacter coli, Campylobacter fetus, Campylobacter jejuni, Campylobacter lari, Capnocytophaga canimorsus, Capnocytophaga cynodegmi, Capnocytophaga gingivalis, Capnocytophaga ochracea, Capnocytophaga sputigena, Chromobacterium violaceum, Comamonas terrigena, Comamonas testosteroni, Corynebacterium diphteriae, Corynebacterium minutissimum, Corynebacterium pseudotuberculosis, Corynebacterium urealyticum, Ehrlichia canis, Ehrlichia chaffeensis, Ehrlichia sennetsu, Eikenella corrodens, Eubacterium lentum, Francisella tularensis, Fusobacterium necrophorum, Fusobacterium nucleatum, Gardnerella vaginalis, Haemophilus influenzae, Helicobacter pylori, Kingella kingae, Methylobacterium extorquens, Methylobacterium mesophilicun, Mycobacterium africanum, Mycobacterium avium, Mycobacterium bovis, Mycobacterium chelonae, Mycobacterium intracellulare, Mycobacterium kanasasii, Mycobacterium leprae, Mycobacterium malmoense, Mycobacterium marinum, Mycobacterium scrofulaceum, Mycobacterium tuberculosis, Mycobacterium xenopi, Neisseria cinerea, Neisseria gonorrhoeae, Neisseria meningitidis, Nocardia asteroides, Nocardia nova, Nocardia octitidiscaviarum, Oligella urethralis, Oligella ureulytica, Peptoniphilus asaccharolyticus, Peptostreptococcus prevotii, Porohyromonas gingivalis, Prevotella buccae, Prevotella buccalis, Prevotella corporis, Prevotella denticola, Prevotella oralis, Rhodococcus equi, Rhodococcus erythropolis, Rhodococcus rhodochrous, Stenotrophomonas maltophilia, Streptobacillus moniliformis, Tropheryma whippley und/oder Weeksella virosa, vorzugsweise in einer physiologischen Probe, detektiert.

Oligonukleotid SEQ ID NO:11 ist vorteilhafterweise spezifisch für die Gattung Actinobacil-Ius, Borrelia, Legionella, Moraxella (Branhamella), Providencia, Pseudomonas und/oder Vibrio ist und detektiert selektiv Actinobacillus actinomycetemcomitans, Actinobacillus equuli, Actinobacillus hominis, Actinobacillus suis, Actinobacillus ureae, Borrelia afzelii, Borrelia burgdorferi, Borrelia garninii, Borrelia hermsii, Borrelia hispanica, Chryseomonas luteola, Legionella dumoffii, Legionella micdadei, Legionella pneumophila, Moraxella (Branhamella) catarrhalis, Moraxella (Branhamella) nonliquefaciens, Moraxella (Branhamella) osloensis, Moraxella (Branhamella) phenylpyruvica, Pediococcus pentosaceus, Porphyromonas asaccharolytica, Providencia alcalifaciens, Providencia rettgeri, Providencia rustigianii, Providencia stuartii, Pseudomonas aeruginosa, Pseudomonas fluorescense, Psychrobacter immobilis, Vibrio cholerae, Vibrio parahaemolyticus und/oder Vibrio vulnificus, vorzugsweise in einer physiologischen Probe.

Von Vorteil erweist sich, dass Oligonukleotid SEQ ID NO:13 und SEQ ID NO:14 spezifisch für die Gattung Actinomyces sind und SEQ ID NO:13 selektiv Actinomyces israelii und SEQ ID NO:14 selektiv Actinomyces odontolyticus und Oligonukleotid SEQ ID NO:15 spezifisch für die Gattung Arcanobacterium ist und selektiv Arcanobacterium haemolyticum, vorzugsweise in einer physiologischen Probe, detektieren.

Oligonukleotid SEQ ID NO:16 bis SEQ ID NO:20 sind vorteilhafterweise spezifisch für die Gattung Bacteroides, wobei SEQ ID NO:16 selektiv Bacteroides eggerthii, SEQ ID NO:17 selektiv Bacteroides fragilis, SEQ ID NO:18 selektiv Bacteroides forsythus, SEQ ID NO:19 selektiv Bacteroides merdae und SEQ ID NO:20 selektiv Bacteroides putredinis, vorzugsweise in einer physiologischen Probe, detektiert.

Oligonukleotid SEQ ID NO:21 und SEQ ID NO:22 sind spezifisch für die Gattung Chlamydiae und SEQ ID NO:21 detektiert selektiv Chlamydiae trachomatis und SEQ ID NO:22 selektiv Chlamydiae pneumoniae, vorzugsweise in einer physiologischen Probe.

Oligonukleotid SEQ ID NO:23 ist spezifisch für die Gattung Fusobacterium und detektiert selektiv Fusobacterium sulci, und Oligonukleotid SEQ ID NO:24 und SEQ ID NO:25 sind spezifisch für die Gattung Leptospira und SEQ ID NO:24 detektiert selektiv Leptospira biflexa und SEQ ID NO:25 Leptospira interrogans, jeweils vorzugsweise in einer physiologischen Probe.

Oligonukleotid SEQ ID NO:26 ist spezifisch für die Gattung Mobiluncus und detektiert selektiv Mobiluncus mulieris und Oligonukleotid SEQ ID NO:27 ist spezifisch für die Gattung Mycoplasma und detektiert selektiv Mycoplasma pneumoniae, jeweils vorzugsweise in einer physiologischen Probe.

Oligonukleotid SEQ ID NO:28 und SEQ ID NO:29 sind vorteilhafterweise spezifisch für die Gattung Peptostreptococcus und SEQ ID NO:28 detektiert selektiv Peptostreptococcus anaerobicus und SEQ ID NO:29 selektiv Peptostreptococcus magnus, vorzugsweise in einer physiologischen Probe.

Von Vorteil erweist sich, dass Oligonukleotid SEQ ID NO:30 spezifisch für die Gattung Porphyromonas ist und selektiv Porphyromonas endodontalis und Oligonukleotid SEQ ID NO:31 spezifisch für die Gattung Rothia ist und selektiv Rothia dentocariosa, vorzugsweise in einer physiologischen Probe, detektiert.

Oligonukleotid SEQ ID NO:32 und SEQ ID NO:33 sind spezifisch für die Gattung Sphingobacterium und SEQ ID NO:32 detektiert selektiv Sphingobacterium multivorum und SEQ ID NO:33 selektiv Sphingobacterium spiritovorum, vorzugsweise in einer physiologischen Probe.

Dabei erweist sich als günstig, dass Oligonukleotid SEQ ID NO:34 spezifisch für die Gattung Treponema ist und selektiv Treponema pallidum und Oligonukleotid SEQ ID NO:35 spezifisch für die Gattung Ureaplasma ist und selektiv Ureaplasma urealyticum, vorzugsweise in einer physiologischen Probe, detektiert.

Die Oligonukleotide der SEQ ID NO:5 bis SEQ ID NO:35 erweisen sich als besonders vorteilhaft, weil dadurch Bakterienarten spezifisch erkannt werden können und zudem eine Zuordnung zu bestimmten Bakteriengattungen erfolgen kann.

Vorteilhaft erweist sich auch, dass die Oligonukleotide SEQ ID NO:5 bis SEQ ID NO:35 Nukleotidsequenzen komplementär zu den erfindungsgemäßen Oligonukleotidsequenzen aufweisen können, wodurch an beide Stränge des DNA-Doppelstranges das Oligonukleotid binden kann und somit jeder der beiden Stränge der DNA-Sequenz kontaminierender Bakterien in einer biologischer Probe erkannt werden kann.

Gemäß einem weiteren Aspekt betrifft die vorliegende Erfindung eine Zusammensetzung umfassend eine Kombination von Oligonukleotiden zum Nachweis von Bakterien-DNA kodierend für eine Sequenz der hochkonservierten 16S rDNA Region, wobei das erste Oligonukleotid mindestens 10 aufeinander folgende Basen der Oligonukleotide SEQ ID NO:5 aufweist und am 3'-Ende mit Fluorescein markiert ist und das weitere Oligonukleotid mindestens 10 aufeinander folgende Basen zumindest eines der Oligonukleotide SEQ ID NO:6 bis SEQ ID NO:35 aufweist und am 5'-Ende mit einem Fluoreszenzfarbstoff markiert ist, wodurch von Vorteil ist, dass das Fluoreszenz-Resonanz-Energie-Transfer (FRET)-Prinzip genutzt werden kann. Die beiden Hybridisierungssonden binden an der gesuchten Ziel-DNA in räumlicher Nähe und das Fluorescein der ersten Hybridisierungssonde überträgt die Energie auf den benachbarten zweiten Fluoreszenzfarbstoff, der nun Fluoreszenz emittiert, deren Intensität in Summe direkt proportional der Menge an Ziel-DNA ist.

Die Realtime-PCR ermöglicht sowohl die Amplifikation als auch Detektion im gleichen geschlossenen Reaktionsgefäß, wodurch sowohl das Risiko der Übertragung einer Kontamination von einem zum anderen Reaktionsgefäß als auch der zeitliche Aufwand, welcher durch das Öffnen und Schließen des Reaktionsgefäßes bzw. durch das Umpipettieren des Amplifikats entstehen, verringert werden. Die erfindungsgemäße Realtime PCR zur Detektion von Bakterien in physiologischen Proben zeichnet sich zudem durch ihre hohe Robustheit und hohe Routinefähigkeit aus. Im Vergleich zu Kultivierungsmethoden gibt es keine signifikanten Unterschiede, wohingegen die Realtime PCR-Methode ein schnelleres Ergebnis als die Kultivierung liefert. Auch Erreger, die sich nicht kultivieren lassen bzw. Erreger, die sehr langsam wachsen, können mittels PCR zeitgerecht und sehr sensitiv detektiert werden.

Durch die Oligonukleotide mit einer Länge von mindestens 10 aufeinander folgenden Basen der Oligonukleotide SEQ ID:1 und SEQ ID NO:2 als Primer wird auf vorteilhafte Weise ermöglicht, dass hochkonservierte Sequenzen von verschiedenen Bakterienstämmen und-arten mit Primern derselben Sequenz amplifiziert werden können.

Vorzugsweise werden Kontrollen, wie eine interne Kontrolle ab der Extraktion und zumindest eine Negativ- und Positivkontrolle, insbesondere während der Amplifikation, mitgeführt. Dadurch können falsch positive und falsch negative Ergebnisse, welche durch eine Funktionsstörung oder Verunreinigung verursacht werden, ausgeschlossen werden.

Vorzugsweise enthält die zumindest eine Positivkontrolle eine Nukleinsäuresequenz von Salmonella choleraesius, Pseudomonas aeruginosa und/oder Staphylococcus epidermidis, wodurch gewährleistet wird, dass bei einem störungsfreien Verfahren in jedem Fall bei den Positivkontrollen ein Signal detektiert werden kann und somit ein Rückschluss auf das Funktionieren der Extraktion, Amplifikation und Detektion zulässig ist.

Dabei ist es besonders vorteilhaft, wenn die Nukleinsäuresequenz der zumindest einen internen Kontrolle mit einem Primerpaar bestehend aus einem Oligonukleotid mit einer Länge von mindestens 10 aufeinander folgenden Basen des Oligonukleotids SEQ ID NO:3 und SEQ ID NO:4 amplifiziert wird, wodurch Nukleinsäuresequenzen der internen Kontrolle nachgewiesen werden, die bereits ab der Extraktion zugesetzt sind und coamplifiziert werden und somit auch als Workflow-Kontrolle dienen. Es kann dadurch beispielsweise auch eine Extraktionsschwankung oder eine unzureichende Amplifikation nachgewiesen werden.

Vorzugsweise werden dabei die Nukleinsäuresequenzen der internen Kontrolle mit einem Oligonukleotid mit einer Länge von mindestens 10 aufeinander folgenden Basen des Oligonukleotids SEQ ID NO: 12 oder deren komplementäre Sequenz hybridisiert, wodurch auch die interne Kontrolle mit demselben Verfahren amplifiziert und insbesondere detektiert werden kann. Durch die Zugabe der internen Kontrolle ab der Extraktion ist es möglich, den gesamten Prozess beginnend bei der Extraktion bis zur Hybridisierung zu überprüfen. Zudem zeichnet sich das Amplifikat der internen Kontrolle durch eine Länge von ca. 60 Basenpaaren aus.

Die Realtime-PCR wird in Kapillaren durchgeführt, wodurch ein weiterer Vorteil dieses Verfahrens ist, dass die PCR-Reaktionsgefäße nach der Amplifikation nicht mehr geöffnet werden müssen, da die Messungen und Quantifizierung nach Beendigung der PCR-Reaktion abgeschlossen sind. Es entfällt somit die teilweise aufwändige Aufbringung der PCR-Produkte auf ein Gel und, was wichtiger ist, es entfällt das Risiko von Carryover-Kontaminationen durch PCR-Produkte, die bei der diagnostischen PCR eine der Hauptkontaminationsquellen sind.

Vorzugsweise erfolgt eine Inkubation der physiologischen Probe mit einer Enzymlösung, insbesondere Lysostaphin-Lysozym-Lösung, vor der Extraktion, um die Sensitivität für den Nachweis schwer aufzuschließender gram-positiver Bakterien zu erhöhen.

Bei der Extraktion werden die Zellen lysiert, bakterielle DNA freigesetzt, die DNA an magnetisierte Partikel gebunden, gereinigt und zuletzt mit einem Puffer eluiert, wodurch standardisierte Verfahren zur Aufreinigung der kontaminierenden Nukleinsäuresequenz für das erfindungsgemäße Verfahren verwendet werden können und somit kostengünstige Methoden zur Verfügung stehen.

Vorzugsweise erfolgt eine Inkubation der physiologischen Probe mit einer Enzymlösung, insbesondere Lysostaphin-Lysozym-Lösung, vor der Extraktion, um die Sensitivität für den Nachweis schwer aufzuschließender gram-positiver Bakterien zu erhöhen.

Dabei ist es besonders günstig eine Nutcleinsäuresequenz aus der hochkonservierten 16S rDNA Region eines Bakteriengenoms zu amplifizieren, um mit dem Einsatz möglichst weniger Reagenzien, insbesondere Primersequenzen, eine möglichst umfangreiche Detektion diverser Bakterienarten zu ermöglichen.

Mit dem erfindungsgemäßen Verfahren können vorteilhafterweise eine Vielzahl unterschiedlicher, physiologischer Proben wie eine Körperflüssigkeit aus einer Gruppe umfassend Blut, Blutfraktionen, Plasma, Knochenmark, Urin, Stuhl, Speichel, Lymphe, Exsudate, Transsudate, Sekrete, Spinalflüssigkeit, Samenflüssigkeit, dispergiertes Gewebe und/oder Flüssigkeiten aus natürlichen oder unnatürlichen Körperhöhlen bzw. Abstriche analysiert werden. Es müssen somit keine oder nur geringe Adaptierungsschritte für Proben jeweils unterschiedlichen Ursprungs durchgeführt werden.

Besonders bevorzugt als physiologische Probe werden mit dem erfindungsgemäßen Verfahren Thrombozyten analysiert. Thrombozytenkonzentrate sind nämlich für den Großteil von Blutbestandteil-assoziierter Sepsis verantwortlich. Die Häufigkeit von bakteriellen Kontaminationen von Thrombozyten ist relativ hoch.

Aufgrund der spezifischen Sonden ist es weiters möglich gram-positive und gram-negative Bakterien zu unterscheiden und mittels der Schmelztemperatur den jeweiligen Bakterienstämmen zuzuordnen bzw. die Bakterienart zu bestimmen.

Vorzugsweise sind im Kit auch weitere Komponenten, wie Magnesiumchloridlösung, Rinderserumalbumin, Puffer und steriles Wasser enthalten, wodurch aufeinander abgestimmte Reagenzien zur Detektion verschiedener Bakterienarten verwendet werden und somit ein Einschleppen von Fremdkontaminationen mit Reagenzien unbekannten Ursprungs vermieden werden kann.

Im speziellen beschreibt die vorliegende Erfindung ein Verfahren zur Detektion von Bakterienkontaminationen in physiologischen Flüssigkeiten.

Die Probenvorbereitung der biologischen Probe erfolgt anhand der automatisierten Extraktionsmethode MagNA Pure Compact Nucleic Acid Isolation Kit - large volume der Fa. Roche Diagnostics, wobei anhand verschiedener Puffer die Zellen aufgeschlossen werden und bakterielle DNA freigesetzt wird. Die DNA wird an magnetisierte Partikel gebunden, gereinigt und letztendlich in einem speziellen Puffer eluiert.

Um eine höhere Extraktionseffizienz zu erreichen und den Aufschluss der Bakterien zu verbessern, wird die Probe vor der Extraktion mit einer speziellen Enzymlösung, einer Lysostaphin-Lysozym-Lösung, inkubiert.

Die Lysostaphin-Lysozym-Lösung besteht aus 100 mg/ml Lysozym und 5 mg/ml Lysostaphin und Wasser bzw. Puffer, vorzugsweise PBS (phosphate buffered saline).

Es wird beispielsweise DNA aus 1000 µl Plasma oder Thrombozytenkonzentrat nach vorgeschalteter Enzyminkubation zur verbesserten Aufschließung der Bakterien isoliert. Die biologische Probe wird mit 10 µl Lysostaphin-Lysozym-Lösung (Konzentration der Stocksolution: 10 g/ml Lysozym und 15 mg/ml Lysostaphin) für 30 Minuten bei 45°C inkubiert. Die freigesetzte DNA wird anschließend an die magnetisierten Partikel gebunden. Nach mehreren Waschschritten wird die DNA mit 50 µl Elutionspuffer eluiert.

Zusätzlich wird ab der Extraktion eine interne Kontrolle (pAW109, Applied Biosystems) mitgeführt. Diese ab der Extraktion mitgeführte interne Kontrolle wird mittels Taqmansonde (Fam/BHQ-1), Oligonukleotid SEQ ID NO:12) detektiert, wobei das angeregte Fam-Molekül erst durch eine Hydrolyse der Taqmansonde Fluoreszenz abgibt und durch die Trennung von Farbstoff und Quencher die emittierte Fluoreszenz gemessen werden kann, wobei die Intensität der emittierten Fluoreszenz direkt proportional der Menge an Ziel-DNA ist.

Die extrahierte DNA wird anschließend in einer Realtime-PCR amplifiziert, wobei die DNA wird dem Mastermix vermengt und am Light Cycler amplifiziert, und mit bakterienspezifischen Sonden detektiert wird.

Die Quantifizierung der PCR-Produkte erfolgt aufgrund der Nutzung von DNA-Farbstoffen. Die Fluoreszenzfarbstoffe lagern sich in die DNA ein, interkalieren bzw. binden an die doppelsträngige DNA, wodurch die Fluoreszenz dieser Farbstoffe ansteigt. Die Zunahme der Target-DNA korreliert daher mit der Zunahme der Fluoreszenz von Zyklus zu Zyklus. Nach abgelaufener PCR kann eine Schmelzkurvenanalyse durchgeführt werden, anhand derer die Spezifität bestimmt werden kann. Bei einer Schmelzkurvenanalyse wird die DNA aufgeschmolzen und dann die Temperatur langsam erhöht. Für die Schmelzkurzvenanalyse wird 10 sec bei 95°C denaturiert und 30 Sekunden auf 50°C abgekühlt und kontinuierlich auf 80°C mit einer Geschwindigkeit von 0,2°C/s erhöht. Bei einer für jede Sequenz spezifischen Temperatur werden die beiden Hybridisierungssonden räumlich voneinander getrennt, wodurch die Fluoreszenz abnimmt und über eine mathematische Ableitung die Schmelztemperatur bestimmt werden kann.

Die Detektion der Bakterienarten erfolgt online mittels Realtime-PCR. Die Realtime-PCR-Technologie erlaubt eine quantitative Echtzeitanalyse der PCR über die Messung von laserinduzierten Fluoreszenzsignalen. In speziellen hochfeinen Kapillaren, vorzugsweise mit einem Fassungsvermögen von 100 µl, wird die Amplifikation und Analyse durchgeführt.

Unter dem Begriff Realtime PCR wird im Rahmen dieser Erfindung eine Vervielfältigungsmethode für Nukleinsäuren, die auf dem Prinzip der herkömmlichen PCR beruht und zusätzlich die Möglichkeit der Quantifizierung bietet, verstanden. In der Literatur werden folgende Bezeichnungen wie Echtzeit- PCR, Fluoreszenz-Hybridisierungs-PCR, LightCycler-PCR, Hybridisation Probe-PCR, FRET-PCR und quantitative PCR, synonym verwendet. Ein großer Vorteil der Realtime-PCR-Technologie ist die verringerte Kontaminationsgefahr. Das PCR-Amplifikat muss nicht mehr auf ein Agarosegel aufgetragen werden, wodurch eine Übertragung von Kontaminationen vermieden werden kann und zudem die Bestätigungsreaktion im PCR-Lauf durch die fluoreszenzmarkierte Hybridisierungssonden integriert wird.

Die Quantifizierung wird mit Hilfe von Fluoreszenzmessungen am Ende bzw. während eines PCR-Zykluses (Realtime) durchgeführt und unterscheidet sich somit von anderen quantitativen PCR-Methoden, die erst nach Ablauf der PCR quantitativ ausgewertet werden (z.B. kompetitive PCR). Die Fluoreszenz nimmt proportional mit der Menge der PCR-Produkte zu, was eine Quantifizierung möglich macht. Die gelelektrophoretische Auftrennung der Fragmente ist nicht nötig, die Daten sind sofort verfügbar und das Kontaminationsrisiko ist somit gering.

Zum PCR-Ansatz werden neben den spezifischen Primern auch zumindest zwei sequenzspezifische Hybridisierungssonden oder eine sequenzspezifische Taqmansonde zugegeben. Die zumindest eine Sonde bindet an eine Nukleinsäuresequenz zwischen den beiden Primern und ist mit zwei verschiedenen Fluoreszenzfarbstoffen markiert. Die Sonde ist vorzugsweise am 3'-Ende mit einem Quencherfarbstoff und am 5'-Ende mit einem fluoreszierenden Reporterfarbstoff markiert. Wenn die intakte Sonde durch Licht einer vordefinierten Wellenlänge angeregt wird, so wird die Fluoreszenzemission des Reporteriarbstoffes durch die räumliche Nähe zu dem Quencherfarbstoff unterdrückt (FRET-Prinzip). Die Stärke der Fluoreszenz des Reporterfarbstoffes ist proportional zur gebildeten DNA-Menge. Die Reporterfluoreszenz wird in vordefinierten Intervallen gemessen, ohne dass das PCR-Reaktionsgefäß geöffnet werden muss und es kann dadurch relativ einfach der Verlauf der PCR-Reaktion verfolgt werden. Innerhalb der PCR-Kurve kann ein geeigneter Punkt für die Quantifizierung gewählt werden.

Für die Amplifikation wird ein Mastermix hergestellt, der eine Lösung aus Enzym (MolTaq 16S), steriles Wasser, Puffer, Magnesiumchlorid, BSA (Rinderserumalbumin), Dinukleotidtriphosphate (dNTPs), Primer und Sonden darstellt. Da das Enzym rekombinant in Escherichia coli hergestellt wird und kein kontaminationsfreies Enzym kommerziell zur Verfügung steht, wird eine spezielle Taq-Polymerase eingesetzt, die nochmals mit PCR-Filter Units der Fa. Millipore aufgereinigt wird.

Beispielsweise besteht der Mastermix pro Probenansatz aus den nachfolgend aufgelisteten Komponenten und wird abschließend gefiltert:

| Komponente | Volumen in µl je Probenansatz |
|---|---|
| H₂O | 11,05 |
| MgCl₂ 25mM | 3 |
| 10x Molzym Puffer | 6,25 |
| BSA 20µg/µl | 0,6 |
| dNTPs 10mM | 1 |
| MolTaq 16S 5U/µl | 0,6 |
| Summe | 22,5µl |

Zudem werden 2,5 µl eines 20x Detection Mix mit den spezifischen Hybridisierungssonden für jeden Probenansatz vorbereitet. In jede Kapillare werden 25 µl des fertigen Mastermixes vorgelegt und 25 µl der biologischen Probe oder der Negativ- bzw. Positivkontrolle beigemengt.

Der Detektion Mix wird als Stock-Lösung hergestellt und umfasst folgende Komponenten:

| Primer/Sonden | Konzentration in PCR [µM] | Stock [µM] | Volumen [µl] für 100µl |
|---|---|---|---|
| SEQ ID NO:1 | 0,3 | 100 | 6 |
| SEQ ID NO:2 | 0,3 | 100 | 6 |
| SEQ ID NO:5 | 0,2 | 50 | 8 |
| SEQ ID NO:6 | 0,2 | 50 | 8 |
| SEQ ID NO:7 | 0,2 | 50 | 8 |
| SEQ ID NO:8 | 0,2 | 50 | 8 |
| SEQ ID NO:9 | 0,2 | 50 | 8 |
| SEQ ID NO:10 | 0,2 | 50 | 8 |
| SEQ ID NO:11 | 0,2 | 50 | 8 |
| SEQ ID NO:3 | 0,2 | 100 | 4 |
| SEQ ID NO:4 | 0,2 | 100 | 4 |
| SEQ ID NO:12 | 0,2 | 50 | 8 |
| ∑ | | | 84 |
| Wasser | | | 16 |
| ∑ | | | 100 |

Die Verwendung der Hybridisierungssonden ermöglicht die spezifische Detektion des PCR-Produkts, unspezifische Produkte und Primerdimere werden nicht detektiert.

Die Hybridisierungssonden SEQ ID NO:5 bis SEQ ID NO:35 sind mit unterschiedlichen, insbesondere fluoreszierenden Farbstoffen, wie z.B. LC 610, LC 640, LC 670, LC 705 und/oder Fluorescein, markiert.

Durch die Amplifikation mit den ausgewählten Primern in der konservierten 16S rDNA Region resultiert ein 466 Basenpaar-PCR-Produkt. Die Wahl der Ziel-DNA-Sequenz hängt von der größtmöglichen Konservierung der Sequenz in Anbetracht der verschiedenen Bakterienarten ab. Die Bakterien-PCR bedient sich der Primer SEQ ID NO: 1 und SEQ ID NO:2 sowie Hybridisierungssonden, die innerhalb einer hoch konservierten Region der 16S rDNA des Bakteriengenoms lokalisiert sind.

Zur Detektion der gram-positiven und gram-negativen Bakterien, sowie zur Unterscheidung der Bakterienarten werden die verschiedenen Hybridisierungssonden SEQ ID NO:5 bis SEQ ID NO:11 und SEQ ID NO:13 bis SEQ ID NO:35, welche mit unterschiedlichen Farbstoffen markiert sind sowie unterschiedliche Schmelztemperaturen aufweisen, verwendet. Die interne Kontrolle wird mit der Sonde SEQ ID NO:12 detektiert.

Als bakterienspezifische Sonden werden Oligonukleotide der SEQ ID NO:6 bis SEQ ID NO:11 und SEQ ID NO:13 bis SEQ ID NO:35 verwendet, wobei im nachfolgenden aufgezeigt wird, welche der Sonden gram-positive bzw. -negative Bakterienarten identifiziert und/oder für welche Bakterienart spezifisch ist.

| Bakterienart | gram | Sequenz 5'-3' | SEQ ID NO |
|---|---|---|---|
| Actinomyces israelii | pos | ACGGTAGCCGGGTTATGAAGCGCCG | 13 |
| Actinomyces odontolyticus | pos | AGGGTAGTGGGTAAGAAGCGCCG | 14 |
| Arcanobacterium haemolyticum | pos | TGAATAAGCGCCGGCTAAGCGCG | 15 |
| Bacteroides eggerthii | neg | ATGTACCGTATGAATAAGGAT | 16 |
| Bacteroides fragilis | neg | ATGTATAATATGAATAAGGAT | 17 |
| Bacteroides forsythus | neg | ATGTACCTTGTGAATAAGCAT | 18 |
| Bacteroides merdae | neg | ATGTACCCTATGAATAAGCAT | 19 |
| Bacteroides putredinis | neg | AAGTATCGTACGAATAAGGAT | 20 |
| Chlamydiae trachomatis | neg | AGCGTACCAGGTAAAGAAGCACCG | 21 |
| Chlamydiae pneumoniae | neg | AGCGTACCGGGTAAAGAAGCACCG | 22 |
| Fusobacterium sulci | neg | TACCCTTGGAGGAAGCCGCGGCTAACTA | 23 |
| Leptospira biflexa | neg | TACCTACCTAAAGCACCGGCTAACTA | 24 |
| Leptospira interrogans | neg | TACCTGCCTAAAGCACCGGCTAACTA | 25 |
| Mobiluncus mulieris | neg | GGTAGCGGGGGAAGAAGCGCCG | 26 |
| Mycoplasma pmeumoniae | neg | CTGTACCATTTTGAATAAGTGACG | 27 |
| Peptostreptococcus anaerobicus | pos | TACCCTGTGAGGAAGCCCCGGCTAACTA | 28 |
| Peptostreptococcus magnus | pos | TACCATAGGAGGAAGCCCCGGCTAAATA | 29 |
| Porphyromonas endodontalis | neg | CATGTACTCTACGAATAAGTATCG | 30 |
| Rothia dentocariosa | pos | ACGGTAGGTGCAGAGAAAGCGCCG | 31 |
| Sphingobacterium multivorum | neg | CTGAATGTACTGGAAGAATAAGGATCG | 32 |
| Sphingobacterium spiritivorum | neg | CTGAATGTACCCAAGAATAAGGATCG | 33 |
| Treponema pallidum | neg | ACGGTAGTCGTGCGAATAAGCCCCG | 34 |
| Ureaplasma urealyticum | neg | ACTGTACCATTTGAATAAGTATCG | 35 |
| | | | |
| | pos | TAGTTAGCCGTGGCTTTCTGGTTAGATA | 6 |
| | pos | TAGTTAGCCGTCGCTTTCTGGTTAGATA | 7 |
| | pos | TAGTTAGCCGTGGCTTTCTGGTTAGGTA | 8 |
| | neg | CGGTGCTTCTTCTGCGAGTAAC | 9 |
| | neg | CGGTGCTTATTCTTTAGGTACCGT | 10 |
| | neg | CGGTGCTTATTCTGTTGGTAACGT | 11 |

Die Oligonukleotide der SEQ ID NO:6 bis SEQ ID NO:11 detektieren, wie in der vorstehenden Tabelle beschrieben, teilweise sowohl gram-positive als auch gram-negative Bakterienarten. Welche Bakterienart sie spezifisch detektieren wird im nachfolgenden beschrieben.

Die Oligonukleotide SEQ ID NO:6 detektieren spezifisch die Gattungen Enterococcus, Kurthia, Lactobacillus und/oder Listeria und selektiv Enterococcus avium, Enterococcus casseliflavus, Enterococcus durans, Enterococcus faecalis, Enterococcus faecium, Enterococcus gallinarum, Enterococcus hirae, Kurthia gibsonii, Kurthia sibirica, Lactobacillus acidophilus, Lactobacillus casei, Lactobacillus fermentum, Lactobacillus plantarum, Lactobacillus salivarius, Listeria monocytogenes, Listeria pyogenes, vorzugsweise in einer physiologischen Probe.

Oligonukleotide der SEQ ID NO:7 detektieren spezifisch die Gattungen Leuconostoc und/oder Streptococcus und selektiv Leuconostoc citreum, Leuconostoc lactis, Streptococcus agalactiae, Streptococcus anginosus, Streptococcus bovis, Streptococcus mitis, Streptococcus pneumoniae, Streptococcus pyogenes, Streptococcus salivarius, Streptococcus sanguinis und/oder Streptococcus sp., vorzugsweise in einer physiologischen Probe.

Die Gattungen Bacillus, Clostridium, Mycoplasma und/oder Staphylococcus werden spezifisch von Oligonukleotid SEQ ID NO:8 detektiert. Mit Oligonukleotid SEQ ID:NO 8 werden selektiv Bacillus anthracis, Bacillus circulans, Bacillus pumilus, Bacillus sphaericus, Bacillus subtilis, Bacteroides capillosus, Brevibacillus laterosporus, Clostridium bifermentans, Clostridium botulinum, Clostridium butyricum, Clostridium clostridioforme, Clostridium difficile, Clostridium novyi, Clostridium perfringens, Clostridium septicum, Clostridium sporogenes, Clostridium tetani, Erysipelothrix rhusiopathiae, Fusobacterium alocis, Gemella haemolysans, Mycoplasma orale, Mycoplasma pulmonis, Mycoplasma buccale, Staphylococcus aureus, Staphylococcus capitis, Staphylococcus cohnii, Staphylococcus epidermidis, Staphylococcus haemolyticus, Staphylococcus hominis, Staphylococcus lugdunensis, Staphylococcus xylosus und/oder Veillonella parvula, vorzugsweise in einer physiologischen Probe, nachgewiesen.

Oligonukleotide SEQ ID NO:9 detektieren spezifisch die Gattungen Acinetobacter, Actinomyces, Aeromonas, Anaerobiospirillum, Bartonella, Brucella, Citrobacter, Enterobacter, Haemophilus, Klebsiella, Kluyvera, Legionella, Pasteurella, Proteus, Rickettsia, Salmonella, Serratia, Shigella, Vibrio, Yersinia, und selektiv Acinetobacter baumannii, Acinetobacter calcoaceticus, Acinetobacter haemolyticus, Acinetobacter johnsonii, Acinetobacter junii, Acinetobacter lwoffii, Actinomyces meyeri, Actinomyces pyogenes, Aeromonas caciae, Aeromonas hydrophila, Aeromonas schubertii, Aeromonas veronii, Agrobacterium radiobacter, Alcaligenes faecalis, Anaerobiospirillum succiniciproducens, Anaerobiospirillum thomasii, Acranobacterium pyogenes, Bartonella bacilliformis, Bartonella henselae, Brucella abortus, Brucella melitensis, Calymmatobacterium granulomatis, Citrobacter amalonaticus, Citrobacter freundii, Coxiella burnetti, Edwardsiella tarda, Enterobacter aerogenes, Enterobacter cloacae, Enterobacter sakazakii, Enterobacter sp., Escherichia coli, Haemophilus aegypticus, Haemophilus aphrophilus, Haemophilus ducreyi, Haemophilus parahaemolyticus, Haemophilus parainfluenzae, Haemophilus paraphrophilus, Haemophilus segnis, Hafnia alvei, Klebsiella oxytoca, Klebsiella pneumoniae, Klebsiella rhinoscleromatis, Kluyvera ascorbata, Kluyvera cryocrescens, Legionella dumoffii, Legionella micdadei, Morganella morganii, Ochrobactrum anthropi, Pantoea agglomerans, Pasteurella gallinarum, Pasteurella pneumotropica, Plesiomonas shigelloides, Propionibacterium acnes, Proteus mirabilis, Proteus penneri, Proteus vulgaris, Pseudomonas putida, Rickettsia akari, Rickettsia australis, Rickettsia conorii, Salmonella choleraesius, Salmonella enterica, Salmonella paratyphi A, Salmonella paratyphi B, paratyphi C, Salmonella typhi, Salmonella typhinurium, Serratia ficaria, Serratia fonticola, Serratia grimesii, Serratia liquefaciens, Serratia marcescens, Serratia rudidaea, Shigella boydii, Shigella dysenteriae, Shigella flexneri, Shigella sonnei, Vibrio alginolyticus, Vibrio hollisae, Wigglesworthia glossinidia, Xanthomonas campestris, Yersinia enterocolitica, Yersinia pestis und/oder

Yersinia pseudotuberculosis, vorzugsweise in einer physiologischen Probe.

Oligonukleotid SEQ ID NO:10 detektiert spezifisch die Gattungen Achromobacter, Actinomadura, Actinomyces, Afipia, Bordetella, Burkholderia, Campylobacter, Capnocytophaga, Comamonas, Corynebacterium, Ehrlichia, Fusobacterium, Methylobacterium, Mycobacterium, Neisseria, Nocardia, Oligella, Prevotella und/oder Rhodococcus und selektiv Achromobacter piechaudii, Achromobacter xylosoxidans, Actinomadura madurae, Actinomadura pelletieri, Actinomyces bovis, Actinomyces naeslundii, Actinomyces viscosus, Afipia broomeae, Afipia felis, Bacteroides gracilis, Bilophila wadsworthia, Bordetella bronchiseptica, Bordetella parapertussis, Bordetella pertussis, Burkholderia cepacia, Burkholderia gladioli, Burkholderia mallei, Burkholderia pseudomallei, Campylobacter coli, Campylobacter fetus, Campylobacter jejuni, Campylobacter lari, Capnocytophaga canimorsus, Capnocytophaga cynodegmi, Capnocytophaga gingivalis, Capnocytophaga ochracea, Capnocytophaga sputigena, Chromobacterium violaceum, Comamonas terrigena, Comamonas testosteroni, Corynebacterium diphteriae, Corynebacterium minutissimum, Corynebacterium pseudotuberculosis, Corynebacterium urealyticum, Ehrlichia canis, Ehrlichia chaffeensis, Ehrlichia sennetsu, Eikenella corrodens, Eubacterium lentum, Francisella tularensis, Fusobacterium necrophorum, Fusobacterium nucleatum, Gardnerella vaginalis, Haemophilus influenzae, Helicobacter pylori, Kingella kingae, Methylobacterium extorquens, Methylobacterium mesophilicun, Mycobacterium africanum, Mycobacterium avium, Mycobacterium bovis, Mycobacterium chelonae, Mycobacterium intracellulare, Mycobacterium kanasasii, Mycobacterium leprae, Mycobacterium malmoense, Mycobacterium marinum, Mycobacterium scrofulaceum, Mycobacterium tuberculosis, Mycobacterium xenopi, Neisseria cinerea, Neisseria gonorrhoeae, Neisseria meningitidis, Nocardia asteroides, Nocardia nova, Nocardia octitidiscaviarum, Oligella urethralis, Oligella ureulytica, Peptoniphilus asaccharolyticus, Peptostreptococcus prevotii, Porohyromonas gingivalis, Prevotella buccae, Prevotella buccalis, Prevotella corporis, Prevotella denticola, Prevotella oralis, Rhodococcus equi, Rhodococcus erythropolis, Rhodococcus rhodochrous, Stenotrophomonas maltophilia, Streptobacillus moniliformis, Tropheryma whippley und/oder Weeksella virosa, vorzugsweise in einer physiologischen Probe.

Die Gattungen Actinobacillus, Borrelia, Legionella, Moraxella (Branhamella), Providencia, Pseudomonas und/oder Vibrio werden spezifisch von Oligonukleotid SEQ ID NO:11 detektiert und weisen selektiv Actinobacillus actinomycetemcomitans, Actinobacillus equuli, Actinobacillus hominis, Actinobacillus suis, Actinobacillus ureae, Borrelia afzelii, Borrelia burgdorferi, Borrelia garninii, Borrelia hermsii, Borrelia hispanica, Chryseomonas luteola, Legionella dumoffii, Legionella micdadei, Legionella pneumophila, Moraxella (Branhamella) catarrhalis, Moraxella (Branhamella) nonliquefaciens, Moraxella (Branhamella) osloensis, Moraxella (Branhamella) phenylpyruvica, Pediococcus pentosaceus, Porphyromonas asaccharolytica, Providencia alcalifaciens, Providencia rettgeri, Providencia rustigianii, Providencia stuartii, Pseudomonas aeruginosa, Pseudomonas fluorescense, Psychrobacter immobilis, Vibrio cholerae, Vibrio parahaemolyticus und/oder Vibrio vulnificus, vorzugsweise in einer physiologischen Probe, nach.

Die Amplifikation der zu analysierenden biologischen Proben und der Negativ-, Positiv- und internen Kontrollen durchläuft folgendes PCR-Programm:

Die biologische Probe wird vorab 4 Minuten bei 95°C denaturiert und es folgen 45 Zyklen, wobei in jedem Zyklus die Denaturierung bei 95°C für 5 Sekunden, das Annealing bei 60°C für 25 Sekunden und die Extension 40 Sekunden bei 72°C erfolgt. Für die Schmelzkurve wird 10 Sekunden bei 95°C denaturiert, 30 Sekunden auf 50°C abgekühlt und kontinuierlich auf 80°C mit einer Geschwindigkeit von 0,2°C/s erhöht.

Während des gesamten Workflows werden Positivkontrollen - nämlich Staphylococcus epidermidis 1000 cfu/ml, Pseudomonas aeruginosa 2000 cfu/ml und Salmonella choleraesius 2000 cfu/ml - und eine Negativkontrolle (humanes Thrombozytenkonzentrat) mitgeführt. Um eine mögliche Inhibition feststellen zu können, wird in jedem Reaktionsansatz pAW 109 cDNA als interne Kontrolle coamplifiziert. Die Nachweisgrenze für die einzelnen Bakterienarten liegt bei > 5cfu/ml.

Die Negativkontrolle und die Positivkontrolle Staphylococcus epidermidis 1000 cfu/ml werden ab der Extraktion mitgeführt und die zwei weiteren Positivkontrollen Salmonella choleraesius 2000 cfu/ml und Pseudomonas aeruginosa 2000 cfu/ml erst ab der Amplifikation.

Für die Herstellung der Positivkontrolle mit Staphylococcus epidermidis werden abzentrifiguiertes Thrombozytenkonzentrat und eine Bakteriensuspesion in einer Konzentration von 1000 cfu/ml in einem Verhältnis von annähernd 4:1 pipettiert.

Für die Herstellung der Positivkontrolle mit Salmonella choleraesius 2000cfu/ml werden beispielsweise Thrombozytenkonzentrat und die Bakteriensuspension in einem Verhältnis von annähernd 2:1 vermischt. Weiters werden 1 ml Positivkontrolle mit 10 ml Lysozym-Lysostaphin-Lösung versetzt, gemischt und vorzugsweise bei 45°C inkubiert.

Die Herstellung der Positivkontrolle von Pseudomonas aeruginosa erfolgt wie jene von Salmonella choleraesius.

Anschließend erfolgt die Extraktion auf MagNa Pure Compact unter der Zugabe der internen Kontrolle pAW109.

Als interne Kontrolle wird GeneAmpRNA pAW109 ab der Extraktion verwendet, die vorerst in cDNA umgeschrieben, anschließend amplifiziert, verdünnt und gereinigt wird, vorzugsweise mit ExoSap und Microcon-Filtration, bevor sie als Kontrolle für klinische Analysen eingesetzt wird. Die interne Kontrolle wird den physiologischen Proben zugesetzt und ab Extraktion mitgeführt. Die interne Kontrolle wird mittels spezifischen Primern - SEQ ID NO:3 und SEQ ID NO:4 - im Reaktionsansatz coamplifiziert und dient als Workflow-Kontrolle. Der für die Amplifikation verwendete Vorwärtsprimer weist zumindest 10 aufeinander folgende Basen der Sequenz SEQ ID NO:1 5'-tcctacgggaggcagcagt-3' und der Rückwärtsprimer 10 aufeinander folgende Basen der Sequenz SEQ ID NO:2 5'- ggactaccagggtatetaatcctgtt-3' auf.

Zur Detektion der internen Kontrolle wird eine Fam/BHQ-1 markierte Taqman-Sonde dem Amplifikationsmix beigemengt, wobei das angeregte Fam-Molekül erst durch eine Hydrolyse der Taqmansonde Fluoreszenz abgibt und durch die Trennung von Farbstoff und Quencher die emittierte Fluoreszenz gemessen werden kann, wobei die Intensität der emittierten Fluoreszenz direkt proportional der Menge an Ziel-DNA ist. Die Sonden SEQ ID NO:6 bis SEQ ID NO:11 und SEQ ID:13 bis SEQ ID NO:35 können ebenfalls als Taqman-Sonden verwendet werden.

Wenn die beiden Fluoreszenzfarbstoffe (z.B. Tamra oder Fam des 5'-Endes und BHQ-1 des 3'-Endes) der Taqman Sonde nahe zusammen sind, wird nach Anregung, beispielsweise durch einen Laser, die Energie des Reporterfarbstoffes auf den Quencherfarbstoff übertragen und dieser emittiert Licht. Während der PCR werden beide Primer mittels Polymerase so lange verlängert, bis sie auf die Sonde treffen. Dort wird die hybridisierte DNA-Sonde vom DNA-Strang gelöst und mit Hilfe mit der 5'-3'- Exonukleaseaktivität der Polymerase abgebaut. Durch die Sondenhydrolyse wird die räumliche Nähe zwischen Reporter und Quencher unterbrochen und eine steigende Fluoreszenz des Reporterfarbstoffs kann gemessen werden. Eine Hydrolyse der Sonde durch die 5'-3'- Exonukleaseaktivität kann nur dann erfolgen, wenn es zu einer sequenzspezifischen Hybridisierung zwischen Sonde und Zielsequenz kommt. Entsprechend der Amplifikation des spezifischen PCR-Fragments steigt das Fluoreszenzsignal an. Dabei ist die Fluoreszenzzunahme dem Zuwachs an PCR-Amplifikaten direkt proportional.

Durch die interne Kontrolle kann eine Extraktionsschwankung oder eine unzureichende Amplifikation festgestellt werden. Das Amplifikat zeichnet sich durch eine Länge von annähernd 60 Basenpaaren aus. Eine Fam-BHQ-1 markierte Taqmansonde hybridisiert mit dem Template. Das Signal wird im Kanal 530 abgelesen und analysiert (wie nachfolgend noch ausführlich beschrieben). Die Sequenz der Fam-BHQ-1 markierten Taqmansonde ist SEQ ID NO:12.

Die Amplifikation der internen Kontrolle erfolgt in einer Lösung bestehend aus Wasser, Mn, Enzymmix, Primer der SEQ ID NO:3 und SEQ ID NO:4 sowie der Sonde der SEQ ID NO:12. Im speziellen betragen die Konzentrationen und Volumina für den Mastermix: 3,5 µl Wasser, 1 µl 50 mM Mn, 7,5 µl 2,7x Enzymmix, je 1 µl 5 mM Primer SEQ ID NO:3 und SEQ ID NO:4 und 1 µl 5 mM Sonde SEQ ID NO:12. 15 µl des Mastermix werden zu 5 µl der positiven Kontrolle pipettiert und revers transkribiert und amplifiziert. Die reverse Transkription erfolgt für 30 Minuten bei 60°C. Zur Amplifikation wird die Probe vorab 8 Minuten bei 95°C denaturiert und anschließend in 45 Zyklen zu je 15 Sekunden bei 95°C, 1 Minute bei 60°C und 1 Minute bei 40°C amplifziert.

Eine Inhibition durch positive Amplifikation der internen Kontrolle kann ausgeschlossen werden.

Die Auswertung über Schmelzkurven erlaubt eine Unterscheidung von gram-positiven und gram-negativen Bakterien und eine Zuweisung zu Bakterienarten anhand der Schmelztemperatur der Hybridisierungssonden von der amplifizierten DNA. Schmelzkurvenanalysen werden am Ende der PCR durchgeführt. Dabei wird eine Temperaturkurve, beginnend bei 50 °C, bis 80 °C durchgeführt und aufgezeichnet. An einem bestimmten Temperaturpunkt lösen sich dabei die Sonden von der bakteriellen DNA und die Fluoreszenz nimmt ab. Durch Integrieren der Fluoreszenz durch die Temperatur entsteht ein Schmelzpeak, der zur Auswertung der Ergebnisse herangezogen werden kann.

Die Beurteilung und Auswertung der Wildtypproben erfolgt ausschließlich mit Schmelzkurvenanalyse mittels eines nach der Validierung festgelegten Cut offs. Durch sehr leicht auftretende bakterielle Kontaminationen während des Workflows bzw. durch das Enzym wird dieser Cut off bei der Schmelzkurvenanalyse bei einem Fluoreszenzsignal von > 0,03 bestimmt. Die Auswertung der internen Kontrolle erfolgt über absolute Quantifikationsanalyse.

Für eine eindeutige Auswertung aller Kanäle muss eine MultiColorDemo-kit Color Compensation importiert werden, um den Crosstalk zwischen den Kanälen auszuschalten.

Nach Abschluss der PCR werden die einzelnen Kanäle wie folgt ausgewertet und die interne Kontrolle und die Positivkontrollen kontrolliert:

| | |
|---|---|
| Kanal 530: | interne Kontrolle pAW109; |
| Kanal 610: | Staphylococcus epidermidis 1000 cfu/ml (gram positiv) |
| | Staphylococcus aureus 1000 cfu/ml (gram positiv) |
| Kanal 640: | Salmonella choleraesius 2000 cfu/ml (gram negativ) |
| Kanal 705: | Pseudomonas aeruginosa 2000 cfu/ml (gram negativ) |

Im Kanal 530 müssen alle Proben einen positiven Kurvenanstieg aufweisen. Im Kanal 610 werden gram-positive Bakterien nachgewiesen. Die Positivkontrolle Staphylococcus epidermidis muss in der Schmelzkurvenanalyse einen eindeutigen Kurvenverlauf mit einem Fluoreszenzwert von ~0,55 aufweisen. Zudem wird dieser Fluoreszenzwert auch als Funktionsüberprüfung des Lysozym-Lysostaphin-Verdaus verwendet.

Gram-negative Bakterien werden im Kanal 640 analysiert und detektiert, wobei Salmonella choleraesius in der Schmelzkurvenanalyse einen eindeutigen Kurvenverlauf mit einem Fluoreszenzwert von ~ 0,6 aufweisen muss.

Auch im Kanal 705 werden gram-negative Bakterien detektiert. Die Positivkontrolle Pseudomonas aeruginosa muss in der Schmelzkurvenanalyse einen eindeutigen Kurvenverlauf mit einem Fluoreszenzwert von ~0,8 zeigen.

Ein deutlicher Schmelzkurvenanstieg einer zu untersuchenden biologischen Probe in einem der 4 Kanäle, der eine Fluoreszenz >0,03 aufweist, gilt als positiv. Solche Proben werden im klinischen Alltag um eine relevante Aussage treffen zu können erneut extrahiert und amplifiziert.

Im nachfolgenden werden exemplarisch die Schmelztemperaturen und Kanäle einzelner Bakterienarten aufgelistet:

| Bakterienart | Typus | Schmelztemperatur | Kanal |
|---|---|---|---|
| Pseudomonas aeruginosa | gram-negativ | 68,5 | 705 |
| Salmonella choleraesius | gram-negativ | 62,5 | 640 |
| Staphylococcus epidermidis | gram-positiv | 68 | 610 |
| Enterococcus faecalis | gram-positiv | 71 | 610 |
| Clostridium sporogenes | gram-positiv | 60,8 | 610 |
| Lactobacillus fermentum | gram-positiv | 64,6 | 610 |
| Staphylococcus aureus | gram-positiv | 67,5 | 610 |
| Bacillus subtilis | gram-positiv | 71,5 | 610 |

Weiters wird auch die Schmelzkurve des Kanals 670 für die Probenanalyse ausgewertet. Die Überprüfung der internen Kontrolle erfolgt im Kanal 530 über die Crossing Points in der Quantification Analyse.

Die Auswertung der Analyse erfolgt über den so genannten CT-Wert (threshold cycle). Der CT-Wert drückt die Zyklenzahl aus, bei der zum ersten Mal ein Anstieg der Reporterfluoreszenz über das Grundrauschen ermittelt wird.

Die Detektion der internen Kontrolle basiert auf dem bereits beschriebenen Taqman-Prinzip, wobei das angeregte Fam-Molekül am 5'-Ende des Oligonukleotids SEQ ID NO:12 erst durch Hydrolyse der Taqman-Sonde das Licht im Kanal 530 abgibt. Nur durch die Trennung von Reporter- und Quencherfarbstoff kann das emittierte Licht gemessen werden. Die Intensität des emittierten Lichtes ist direkt proportional der Menge an Ziel-DNA.

Die Positivkontrollen in den verschiedenen Kanälen zeigen einen Funktionsgraphen mit einem Peak bei folgenden Temperaturen:

| Bakterienart | Kanal | Schmelztemperatur in °C |
|---|---|---|
| Staphylococcus aureus | 610 | 67,5 |
| Escherichia coli | 640 | 62 |
| Pseudomonas aeruginosa | 705 | 68,5 |

Abschließend sei angemerkt, dass auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen können.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten der Zusammensetzung, wobei an dieser Stelle bemerkt sei, dass die Erfindung nicht auf die speziell dargestellten Ausführungsbeispiele derselben eingeschränkt ist, sondern vielmehr auch diverse Kombinationen der einzelnen Ausführungsbeispiele untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsbeispiele, die durch Kombinationen einzelner Details der dargestellten und beschriebenen Ausführungsvariante möglich sind, vom Schutzumfang mit umfasst.

## Patentansprüche

1. Verfahren zur Detektion von zumindest einer Bakterienkontamination und Unterscheidung in gram-positive und/oder gram-negative Bakterien, vorzugsweise einer physiologischen Probe umfassend die Schritte i) Extraktion der Nukleinsäure, insbesondere Bakterien-DNA, ii) Amplifikation mit Primern mit einer Länge von mindestens 10 aufeinander folgenden Basen der Oligonukleotide SEQ ID:1 1 und SEQ ID NO:2 und Detektion mit Oligonukleotiden, insbesondere fluoreszenzmarkierten Oligonukleotiden, mit mindestens 10 aufeinander folgende Basen einer Sequenz SEQ ID NO:5, welche am 3'-Ende mit Fluorescein markiert ist und zumindest einer Sequenz ausgewählt aus einer Gruppe umfassend SEQ ID NO:6 bis SEQ ID NO:11 als Hybridisierungssonden, welche am 5'-Ende mit einem Fluoreszenzfarbstoff markiert sind, vorzugsweise in einer Realtime-PCR und iii) Auswertung über Schmelzkurvenanalyse.

2. Verfahren nach Anspruch 1, **dadurch gekennzeichnet, dass** bei der Extraktion die Zellen lysiert werden, bakterielle DNA freigesetzt wird, an magnetisierte Partikel bindet, gereinigt und zuletzt mit einem Puffer eluiert wird.

3. Verfahren nach Anspruch 1 oder 2, **dadurch gekennzeichnet, dass** eine Inkubation der physiologischen Probe mit einer Enzymlösung, insbesondere Lysostaphin-Lysozym-Lösung, vor der Extraktion erfolgt.

4. Verfahren nach einem der Ansprüche 1 bis 3, **dadurch gekennzeichnet, dass** eine Nukleinsäuresequenz aus der hochkonservierten 16S rDNA Region eines Bakteriengenoms amplifiziert wird.

5. Verfahren nach einem der Ansprüche 1 bis 4, **dadurch gekennzeichnet, dass** Kontrollen, wie eine interne Kontrolle vorzugsweise ab der Extraktion, eine Negativ- und zumindest eine Positivkontrolle, mitgeführt werden.

6. Verfahren nach Anspruch 5, **dadurch gekennzeichnet, dass** die zumindest eine Positivkontrolle eine Nukleinsäuresequenz von Salmonella choleraesius, Pseudomonas aeruginosa und/oder Staphylococcus epidermidis enthält.

7. Verfahren nach Anspruch 5 oder 6, **dadurch gekennzeichnet, dass** die Nukleinsäuresequenz der zumindest einen internen Kontrolle mit einem Primerpaar bestehend aus einem Oligonukleotid mit einer Länge von mindestens 10 aufeinander folgenden Basen des Oligonukleotids SEQ ID NO:3 und SEQ ID NO:4 amplifiziert wird.

8. Verfahren nach einem der Ansprüche 5 bis 7, **dadurch gekennzeichnet, dass** die Nukleinsäuresequenzen der internen Kontrolle mit einem Oligonukleotid mit einer Länge von mindestens 10 aufeinander folgenden Basen des Oligonukleotids SEQ ID NO: 12 hybridisiert werden.

9. Verfahren nach einem der Ansprüche 1 bis 8, **dadurch gekennzeichnet, dass** die Primer bzw. Oligonukleotide eine komplementäre Nukleotidsequenz aufweisen.

10. Verfahren nach einem der Ansprüche 1 bis 9, **dadurch gekennzeichnet, dass** die Real-time-PCR in Kapillaren durchgeführt wird.

11. Verfahren nach einem der Ansprüche 1 bis 10, **dadurch gekennzeichnet, dass** als physiologische Probe eine Körperflüssigkeit aus einer Gruppe umfassend Blut, Blutfraktionen, Plasma, Knochenmark, Urin, Stuhl, Speichel, Lymphe, Exsudate, Transsudate, Sekrete, Spinalflüssigkeit, Samenflüssigkeit, dispergiertes Gewebe und/oder Flüssigkeiten aus natürlichen oder unnatürlichen Körperhöhlen bzw. Abstriche ausgewählt wird.

12. Verfahren nach einem der Ansprüche 1 bis 11, **dadurch gekennzeichnet, dass** als physiologische Probe Blutbestandteile, wie z.B. Thrombozyten, verwendet werden.

13. Verfahren nach einem der Ansprüche 1 bis 12, **dadurch gekennzeichnet, dass** die Bakterienarten Enterococcus avium, Enterococcus casseliflavus, Enterococcus durans, Enterococcus faecalis, Enterococcus faecium, Enterococcus gallinarum, Enterococcus hirae, Kurthia gibsonii, Kurthia sibirica, Lactobacillus acidophilus, Lactobacillus casei, Lactobacillus fermentum, Lactobacillus plantarum, Lactobacillus salivarius, Listeria monocytogenes, Listeria pyogenes, Leuconostoc citreum, Leuconostoc lactis, Streptococcus agalactiae, Streptococcus anginosus, Streptococcus bovis, Streptococcus mitis, Streptococcus pneumoniae, Streptococcus pyogenes, Streptococcus salivarius, Streptococcus sanguinis, Streptococcus sp., Bacillus anthracis, Bacillus circulans, Bacillus pumilus, Bacillus sphaericus, Bacillus subtilis, Bacteroides capillosus, Brevibacillus laterosporus, Clostridium bifermentans, Clostridium botulinum, Clostridium butyricum, Clostridium clostridioforme, Clostridium difficile, Clostridium novyi, Clostridium perfringens, Clostridium septicum, Clostridium sporogenes, Clostridium tetani, Erysipelothrix rhusiopathiae, Fusobacterium alocis, Gemella haemolysans, Mycoplasma orale, Mycoplasma pulmonis, Mycoplasma buccale, Staphylococcus aureus, Staphylococcus capitis, Staphylococcus cohnii, Staphylococcus epidermidis, Staphylococcus haemolyticus, Staphylococcus hominis, Staphylococcus lugdunensis, Staphylococcus xylosus, Veillonella parvula, Acinetobacter baumannii, Acinetobacter calcoaceticus, Acinetobacter haemolyticus, Acinetobacter johnsonii, Acinetobacter junii, Acinetobacter lwoffii, Actinomyces meyeri, Actinomyces pyogenes, Aeromonas caciae, Aeromonas hydrophila, Aeromonas schubertii, Aeromonas veronii, Agrobacterium radiobacter, Alcaligenes faecalis, Anaerobiospirillum succiniciproducens, Anaerobiospirillum thomasii, Acranobacterium pyogenes, Bartonella bacilliformis, Bartonella henselae, Brucella abortus, Brucella melitensis, Calymmatobacterium granulomatis, Citrobacter amalonaticus, Citrobacter freundii, Coxiella burnetti, Edwardsiella tarda, Enterobacter aerogenes, Enterobacter cloacae, Enterobacter sakazakii, Enterobacter sp., Escherichia coli, Haemophilus aegypticus, Haemophilus aphrophilus, Haemophilus ducreyi, Haemophilus parahaemolyticus, Haemophilus parainfluenzae, Haemophilus paraphrophilus, Haemophilus segnis, Hafnia alvei, Klebsiella oxytoca, Klebsiella pneumoniae, Klebsiella rhinoscleromatis, Kluyvera ascorbata, Kluyvera cryocrescens, Legionella dumoffii, Legionella micdadei, Morganella morganii, Ochrobactrum anthropi, Pantoea agglomerans, Pasteurella gallinarum, Pasteurella pneumotropica, Plesiomonas shigelloides, Propionibacterium acnes, Proteus mirabilis, Proteus penneri, Proteus vulgaris, Pseudomonas putida, Rickettsia akari, Rickettsia australis, Rickettsia conorii, Salmonella choleraesius, Salmonella enterica, Salmonella paratyphi A, Salmonella paratyphi B, paratyphi C, Salmonella typhi, Salmonella typhinurium, Serratia ficaria, Serratia fonticola, Serratia grimesii, Serratia liquefaciens, Serratia marcescens, Serratia rudidaea, Shigella boydii, Shigella dysenteriae, Shigella flexneri, Shigella sonnei, Vibrio alginolyticus, Vibrio hollisae, Wigglesworthia glossinidia, Xanthomonas campestris, Yersinia enterocolitica, Yersinia pestis, Yersinia pseudotuberculosis, Achromobacter piechaudii, Achromobacter xylosoxidans, Actinomadura madurae, Actinomadura pelletieri, Actinomyces bovis, Actinomyces naeslundii, Actinomyces viscosus, Afipia broomeae, Afipia felis, Bacteroides gracilis, Bilophila wadsworthia, Bordetella bronchiseptica, Bordetella parapertussis, Bordetella pertussis, Burkholderia cepacia, Burkholderia gladioli, Burkholderia mallei, Burkholderia pseudomallei, Campylobacter coli, Campylobacter fetus, Campylobacter jejuni, Campylobacter lari, Capnocytophaga canimorsus, Capnocytophaga cynodegmi, Capnocytophaga gingivalis, Capnocytophaga ochracea, Capnocytophaga sputigena, Chromobacterium violaceum, Comamonas terrigena, Comamonas testosteroni, Corynebacterium diphteriae, Corynebacterium minutissimum, Corynebacterium pseudotuberculosis, Corynebacterium urealyticum, Ehrlichia canis, Ehrlichia chaffeensis, Ehrlichia sennetsu, Eikenella corrodens, Eubacterium lentum, Francisella tularensis, Fusobacterium necrophorum, Fusobacterium nucleatum, Gardnerella vaginalis, Haemophilus influenzae, Helicobacter pylori, Kingella kingae, Methylobacterium extorquens, Methylobacterium mesophilicun, Mycobacterium africanum, Mycobacterium avium, Mycobacterium bovis, Mycobacterium chelonae, Mycobacterium intracellulare, Mycobacterium kanasasii, Mycobacterium leprae, Mycobacterium malmoense, Mycobacterium marinum, Mycobacterium scrofulaceum, Mycobacterium tuberculosis, Mycobacterium xenopi, Neisseria cinerea, Neisseria gonorrhoeae, Neisseria meningitidis, Nocardia asteroides, Nocardia nova, Nocardia, octitidiscaviarum, Oligella urethralis, Oligella ureulytica, Peptoniphilus asaccharolyticus, Peptostreptococcus prevotü, Porohyromonas gingivalis, Prevotella buccae, Prevotella buccalis, Prevotella corporis, Prevotella denticola, Prevotella oralis, Rhodococcus equi, Rhodococcus erythropolis, Rhodococcus rhodochrous, Stenotrophomonas maltophilia, Streptobacillus moniliformis, Tropheryma whippley, Weeksella virosa, Actinobacillus actinomycetemcomitans, Actinobacillus equuli, Actinobacillus hominis, Actinobacillus suis, Actinobacillus ureae, Borrelia afzelii, Borrelia burgdorferi, Borrelia garninii, Borrelia hermsii, Borrelia hispanica, Chryseomonas luteola, Legionella dumoffii, Legionella micdadei, Legionella pneumophila, Moraxella (Branhamella) catarrhalis, Moraxella (Branhamella) nonliquefaciens, Moraxella (Branhamella) osloensis, Moraxella (Branhamella) phenylpyruvica, Pediococcus pentosaceus, Porphyromonas asaccharolytica, Providencia alcalifaciens, Providencia rettgeri, Providencia rustigianii, Providencia stuartii, Pseudomonas aeruginosa, Pseudomonas fluorescense, Psychrobacter immobilis, Vibrio cholerae, Vibrio parahaemolyticus, Vibrio vulnificus, Actinomyces israelii, Actinomyces odontolyticus, Arcanobacterium haemolyticum, Bacteroides eggerthii, Bacteroides fragilis, Bacteroides forsythus, Bacteroides merdae, Bacteroides putredinis, Chlamydiae trachomatis, Chlamydiae pneumoniae, Fusobacterium sulci, Leptospira biflexa, Leptospira interrogans, Mobiluncus mulieris, Mycoplasma pneumoniae, Peptostreptococcus anaerobicus, Peptostreptococcus magnus, Porphyromonas endodontalis, Rothia dentocariosa, Sphingobacterium multivorum, Sphingobacterium spiritovorum, Treponema pallidum und/oder Ureaplasma urealyticum mittels Schmelzkurvenanalyse detektiert werden.

14. Verfahren nach einem der Ansprüche 1 bis 13, **dadurch gekennzeichnet, dass** die Bestimmung der Bakterienarten mittels Schmelzkurvenanalyse durch Überschreitung eines durch Evaluierung vordefinierten Cut-Off Wertes erfolgt.

15. Kit zum Nachweis von Bakterien enthaltend wenigstens 10 aufeinander folgende Basen der Oligonukleotide SEQ ID:1 und SEQ ID NO:2 bzw. SEQ ID:3 und SEQ ID NO:4, sowie 10 aufeinander folgende Basen des Oligonukleotids SEQ ID NO:5, welches am 3'-Ende mit Fluorescein markiert ist und zumindest einer Sequenz ausgewählt aus einer Gruppe umfassend SEQ ID NO:6 bis SEQ ID NO:11 als Hybridisierungssonden, welche am 5'-Ende mit einem Fluoreszenzfarbstoff markiert ist, und gegebenenfalls eine thermostabile DNA-Polymerase und dNTP-Lösungen.

## Claims

1. Method of detecting at least one bacterial contamination, preferably of a physiological sample, and distinguishing between gram-positive and/or gram-negative bacteria, comprising the steps of i) extraction of the nucleic acid, in particular bacterial DNA, ii) amplification by means of primers with a length of at least 10 consecutive bases of the oligonucleotides SEQ ID:1 and SEQ ID NO:2 and detection by means of oligonucleotides, in particular fluorescence-marked oligonucleotides, with at least 10 consecutive bases of a sequence SEQ ID NO:5 which is marked at the 3'-end with fluorescence, and at least one sequence selected from a group comprising SEQ ID NO:6 to SEQ ID NO:11 constituting hybridization probes, marked with a fluorescent dye at the 5' end, preferably in real-time PCR, and iii) evaluation by fusion curve analysis.

2. Method as claimed in claim 1, **characterised in that**, during extraction, the cells are subjected to lysis, bacterial DNA is released and bound to magnetised particles, cleaned and finally eluted by means of a buffer.

3. Method as claimed in claim 1 or 2, **characterised in that** the physiological sample is incubated with an enzyme solution, in particular Lysostasphin-Lysozyme solution, prior to extraction.

4. Method as claimed in one of claims 1 to 3, **characterised in that** a nucleic acid sequence from the highly conserved 16S rDNA region of a bacterial genome is amplified.

5. Method as claimed in one of claims 1 to 4, **characterised in that** controls are employed, such as an internal control preferably from the extraction, a negative and at least one positive control.

6. Method as claimed in claim 5, **characterised in that** the at least one positive control contains a nucleic acid sequence of Salmonella choleraesius, Pseudomonas aeruginosa and/or Staphylococcus epidermidis.

7. Method as claimed in claim 5 or 6, **characterised in that** the nucleic acid sequence of the at least one internal control is amplified with a primer pair consisting of an oligonucleotide with a length of at least 10 consecutive bases of the oligonucleotide SEQ ID NO:3 and SEQ ID NO:4.

8. Method as claimed in one of claims 5 to 7, **characterised in that** the nucleic acid sequences of the internal control are hybridized with an oligonucleotide with a length of at least 10 consecutive bases of the oligonucleotide SEQ ID NO:12.

9. Method as claimed in one of claims 1 to 8, **characterised in that** the primers or oligonucleotides have a complementary nucleotide sequence.

10. Method as claimed in one of claims 1 to 9, **characterised in that** the real-time PCR is run in capillaries.

11. Method as claimed in one of claims 1 to 10, **characterised in that** a body fluid from a group comprising blood, blood fractions, plasma, bone marrow, urine, stool, saliva, lymphs, exudates, transudates, secretions, spinal fluid, semen, dispersed tissue and fluids form natural or unnatural body cavities or swabs are selected as a biological sample.

12. Method as claimed in one of claims 1 to 11, **characterised in that** blood components such as thrombocytes, for example, are selected as a biological sample.

13. Method as claimed in one of claims 1 to 12, **characterised in that** the bacterium types detected by means of fusion curve analysis are Enterococcus avium, Enterococcus casseliflavus, Enterococcus durans, Enterococcus faecalis, Enterococcus faecium, Enterococcus gallinarum, Enterococcus hirae, Kurthia gibsonii, Kurthia sibirica, Lactobacillus acidophilus, Lactobacillus casei, Lactobacillus fermentum, Lactobacillus plantarum, Lactobacillus salivarius, Listeria pyogenes, Leuconostoc citreum, Leuconostoc lactis, Streptococcus agalactiae, Streptococcus anginosus, Streptococcus bovis, Streptococcus mitis, Streptococcus pneumoniae, Streptococcus pyogenes, Streptococcus salivarius, Streptococcus sanguinis, Streptococcus sp., Bacillus anthracis, Bacillus circulans, Bacillus pumilus, Bacillus sphaericus, Bacillus subtilis, Bacteroides capillosus, Brevibacillus laterosporus, Clostridium bifermentans, Clostridium botulinum, Clostridium butyricum, Clostridium clostridioforme, Clostridium difficile, Clostridium novyi, Clostridium perfringens, Clostridium septicum, Clostridium sporogenes, Clostridium tetani, Erysipelothrix rhusiopathiae, Fusobacterium alocis, Gemella haemolysans, Mycoplasma orale, Mycoplasma pulmonis, Mycoplasma buccale, Staphylococcus aureus, Staphylococcus capitis, Staphylococcus cohnii, Staphylococcus epidermidis, Staphylococcus haemolyticus, Staphylococcus hominis, Staphylococcus lugdunensis, Staphylococcus xylosus, Veillonella parvula, Acinetobacter baumannii, Acinetobacter calcoaceticus, Acinetobacter haemolyticus, Acinetobacter johnsonii, Acinetobacter junii, Acinetobacter Iwoffii, Actinomyces meyeri, Actinomyces pyogenes, Aeromonas caciae, Aeromonas hydrophila, Aeromonas schubertii, Aeromonas veronii, Agrobacterium radiobacter, Alcaligenes faecalis, Anaerobiospirillum succiniciproducens, Anaerobiospirillum thomasii, Acranobacterium pyogenes, Bartonella bacilliformis, Bartonella henselae, Brucella abortus, Brucella melitensis, Calymmatobacterium granulomatis, Citrobacter amalonaticus, Citrobacter freundii, Coxiella burnetti, Edwardsiella tarda, Enterobacter aerogenes, Enterobacter cloacae, Enterobacter sakazakii, Enterobacter sp., Escherichia coli, Haemophilus segypticus, Haemophilus aphrophilus, Haemophilus ducreyi, Haemophilus parahaemolyticus, Haemophilus parainfluenzae, Haemophilus paraphrophilus, Haemophilus segnis, Hafnia alvei, Klebsiella oxytoca, Klebsiella pneumoniae, Klebsiella rhinoscleromatis, Kluyvera ascorbata, Kluyvera cryocrescens, Legionella dumoffii, Legionella micdadei, Morganella morganii, Ochrobactrum anthropi, Pantoea agglomerans, Pasteurella gallinarum, Pasteurella pneumotropica, Plesiomonas shigelloides, Propionibacterium acnes, Proteus mirabilis, Proteus penneri, Proteus vulgaris, Pseudomonas putida, Rickettsia akari, Rickettsia australis, Rickettsia conorii, Salmonella choleraesius, Salmonella enterica, Salmonella paratyphi A, Salmonella paratyphi B, paratyphi C, Salmonella typhi, Salmonella typhinurium, Serratia ficaria, Serratia fonticola, Serratia grimesii, Serratia liquefaciens, Serratia marcescens, Serratia rudidaea, Shigella boydii, Shigella dysenteriae, Shigella flexneri, Shigella sonnei, Vibrio alginolyticus, Vibrio hollisae, Wigglesworthia glossinidia, Xanthomonas campestris, Yersinia enterocolitica, Yersinia pestis, Yersinia pseudotuberculosis, Achromobacter piechaudii, Achromobacter xylosoxidans, Actinomadura madurae, Actinomadura pelletieri, Actinomyces bovis, Actinomyces naeslundii, Actinomyces viscosus, Afipia broomeae, Afipia felis, Bacteroides gracilis, Bilophila wadsworthia, Bordetella bronchiseptica, Bordetella parapertussis, Bordetella pertussis, Burkholderia cepacia Methylobacteriumh8ilieun, Burkholderia gladioli, Burkholderia mallei, Burkholderia pseudomallei, Campylobacter coli, Campylobacter fetus, Campylobacter jejuni, Campylobacter lari, Capnocytophaga canimorsus, Capnocytophaga cynodegmi, Capnocytophaga gingivalis, Capnocytophaga ochracea, Capnocytophaga sputigena, Chromobacterium violaceum, Comamonas terrigena, Comamonas testosteroni, Corynebacterium diphteriae, Corynebacterium minutissimum, Corynebacterium pseudotuberculosis, Corynebacterium urealyticum, Ehrlichia canis, Ehrlichia chaffeensis, Ehrlichia sennetsu, Eikenella corrodens, Eubacterium lentum, Francisella tularensis, Fusobacterium necrophorum, Fusobacterium nucleatum, Gardnerella vaginalis, Haemophilus influenzae, Heliobacter pylori, Kingella kingae, Methylobacterium extorquens, Methylobacterium mesophilicun, Mycobacterium africanum, Mycobacterium avium, Mycobacterium bovis, Mycobacterium chelonae, Mycobacterium intracellulare, Mycobacterium kanasasii, Mycobacterium leprae, Mycobacterium malmoense, Mycobacterium marinum, Mycobacterium scrofulaceum, Mycobacterium tuberculosis, Mycobacterium xenopi, Neisseria cinerea, Neisseria gonorrhoeae, Neisseria meningitidis, Nocardia asteroides, Nocardia nova, Nocardia octitidiscaviarum, Oligella urethralis, Oligella ureulytica, Peptoniphilus asaccharolyticus, Peptostreptococcus prevotii, Porohyromonas gingivalis, Prevotella buccae, Prevotella buccalis, Prevotella corporis, Prevotella denticola, Prevotella oralis, Rhodococcus equi, Rhodococcus erythropolis, Rhodococcus rhodochrous, Stenotrophomonas maltophilia, Streptobacillus moniliformis, Tropheryma whippley, Weeksella virosa, Actinobacillus actinomycetemcomitans, Actinobacillus equuli, Actinobacillus hominis, Actinobacillus suis, Actinobacillus ureae, Borrelia afzelii, Borrelia burgdorferi, Borrelia garninii, Borrelia hermsii, Borrelia hispanica, Chryseomonas luteola, Legionella dumoffii, Legionella micdadei, Legionella pneumophila, Moraxella (Branhamella) catarrhalis, Moraxella (Branhamella) nonliquefaciens, Moraxella (Branhamella) osloensis, Moraxella (Branhamella) phenylpyruvica, Pediococcus pentosaceus, Porphyromonas asaccharolytica, Providencia alcalifaciens, Providencia rettgeri, Providencia rustigianii, Providencia stuartii, Pseudomonas aeruginosa, Pseudomonas fluorescense, Psychrobacter immobilis, Vibrio cholerae, Vibrio parahaemolyticus, Vibrio vulnificus, Actinomyces israelii, Actinomyces odontolyticus, Arcanobacterium haemolyticum, Bacteroides eggerthii, Bacteroides fragilis, Bacteroides forsythus, Bacteroides merdae, Bacteroides putredinis, Chlamydiae trachomatis, Chlamydiae pneumoniae, Fusobacterium sulci, Leptospira biflexa, Leptospira interrogans, Mobiluncus mulieris, Mycoplasma pneumoniae, Peptostreptococcus anaerobicus, Peptostreptococcus magnus, Porphyromonas endodontalis, Rothia dentocariosa, Sphingobacterium multivorum, Sphingobacterium spiritovorum, Treponema pallidum and/or Ureaplasma urealyticum.

14. Method as claimed in one of claims 1 to 13, **characterised in that** the bacterium types are detected by means of fusion curve analysis when a cut-off value previously defined by evaluation is exceeded.

15. Kit for detecting bacteria containing at least 10 consecutive bases of the oligonucleotides SEQ ID:1 and SEQ ID NO:2 respectively SEQ ID:3 and SEQ ID NO:4, as well as 10 consecutive bases of the oligonucleotide SEQ ID NO:5 which is marked with fluorescence at the 3'-end and at least one sequence selected from a group comprising SEQ ID NO:6 to SEQ ID NO:11 constituting hybridization probes, marked with a fluorescent dye at the 5' end, and optionally a thermostable DNA polymerase and dNTP solutions.

## Revendications

1. Procédé pour la détection d'au moins une contamination bactérienne et pour la distinction entre les bactéries Gram positif et les bactéries Gram négatif, de préférence d'un échantillon physiologique, comprenant les étapes i) extraction de l'acide nucléique, en particulier de l'ADN bactérien, ii) amplification avec des amorces d'une longueur d'au moins 10 bases consécutives des oligonucléotides SEQ ID:1 et SEQ ID NO:2 et détection avec des oligonucléotides, en particulier des oligonucléotides marqués par fluorescence, avec au moins 10 bases consécutives d'une SEQ ID NO:5, laquelle est marquée à l'extrémité 3' à la fluorescéine, et au moins une séquence choisie parmi un groupe comprenant les SEQ ID NO:6 à SEQ ID NO:11 comme sondes d'hybridation, lesquelles sont marquées à l'extrémité 5' par un colorant fluorescent, de préférence dans une réaction PCR en temps réel, et iii) évaluation par l'intermédiaire de l'analyse de courbes de fusion.

2. Procédé selon la revendication 1, **caractérisé en ce que**, lors de l'extraction, les cellules sont lysée, l'ADN bactérien est libéré, s'attache à des particules magnétisées, est purifié et finalement élué par une solution tampon.

3. Procédé selon la revendication 1 ou 2, **caractérisé en ce qu'**une incubation de l'échantillon physiologique avec une solution enzymatique, en particulier une solution de lysostaphine-lysozyme, s'effectue avant l'extraction.

4. Procédé selon l'une quelconque des revendications 1 à 3, **caractérisé en ce qu'**une séquence d'acide nucléique provenant de la région hautement conservée ADN-r 16S d'un génome bactérien est amplifiée.

5. Procédé selon l'une quelconque des revendications 1 à 4, **caractérisé en ce que** des contrôles tels un contrôle interne, de préférence à partir de l'extraction, un contrôle négatif et au moins un contrôle positif, sont conduits en même temps.

6. Procédé selon la revendication 5, **caractérisé en ce que** ledit au moins un contrôle positif contient une séquence d'acides nucléiques de Salmonella choleraesius, Pseudomonas aeruginosa et/ou Spaphylococcus epidermis.

7. Procédé selon la revendication 5 ou 6, **caractérisé en ce que** la séquence d'acides nucléiques dudit au moins un contrôle interne est amplifiée par une paire d'amorces, consistant en un oligonucléotide d'une longueur d'au moins 10 bases consécutives de l'oligonucléotide SEQ ID NO:3 et SEQ ID NO:4.

8. Procédé selon l'une quelconque des revendications 5 à 7, **caractérisé en ce que** les séquences d'acides nucléiques du contrôle interne sont hybridées avec un oligonucléotide d'une longueur d'au moins 10 bases consécutives de l'oligonucléotide SEQ ID NO:12.

9. Procédé selon l'une quelconque des revendications 1 à 8, **caractérisé en ce que** les amorces, respectivement les oligonucléotides, comportent une séquence de nucléotides complémentaires.

10. Procédé selon l'une quelconque des revendications 1 à 9, **caractérisé en ce que** la réaction PCR en temps réel est effectuée dans des capillaires.

11. Procédé selon l'une quelconque des revendications 1 à 10, **caractérisé en ce que** comme échantillon physiologique est choisi un liquide corporel du groupe comprenant le sang, des fractions du sang, le plasma, la moelle osseuse, l'urine, les fèces, la salive, la lymphe, les exsudats, les transsudats, les sécrétions, le liquide cérébro-spinal, le liquide séminal, du tissus dispersé et/ou des liquides ou des frottis provenant de cavités corporelles naturelles ou non naturelles.

12. Procédé selon l'une quelconque des revendications 1 à 11, **caractérisé en ce que** des composants du sang, comme par exemple des thrombocytes, sont utilisés comme échantillon physiologique.

13. Procédé selon l'une quelconque des revendications 1 à 12, **caractérisé en ce que** les genres de bactéries Enterococcus avium, Enterococcus casseliflavus, Enterococcus durans, Enterococcus faecalis, Enterococcus faecium, Enterococcus gallinarum, Enterococcus hirae, Kurthia gibsonii, Kurthia sibirica, Lactobacillus acidophilus, Lactobacillus casei, Lactobacillus fermentum, Lactobacillus plantarum, Lactobacillus salivarius, Listeria monocytogenes, Listeria pyogenes, Leuconostoc citreum, Leuconostoc lactis, Streptococcus agalactiae, Streptococcus anginosus, Streptococcus bovis, Streptococcus mitis, Streptococcus pneumoniae, Streptococcus pyogenes, Streptococcus salivarius, Streptococcus sanguinis, Streptococcus sp., Bacillus anthracis, Bacillus circulans, Bacillus pumilus, Bacillus sphaericus, Bacillus subtilis, Bacteroides capillosus, Brevibacillus laterosporus, Clostridium bifermentans, Clostridium botulinum, Clostridium butyricum, Clostridium clostridioforme, Clostridium difficile, Clostridium novyi, Clostridium perfringens, Clostridium septicum, Clostridium sporogenes, Clostridium tetani, Erysipelothrix rhusiopathiae, Fusobacterium alocis, Gemella haemolysans, Mycoplasma orale, Mycoplasma pulmonis, Mycoplasma buccale, Staphylococcus aureus, Staphylococcus capitis, Staphylococcus epidermidis, Staphylococcus haemolyticus, Staphylococcus hominis, Staphylococcus lugdunensis, Staphylococcus xylosus, Veillonella parvula, Acinetobacter baumannii, Acinetobacter calcoaceficus, Acinetobacter haemolyticus, Acinetobacter johnsonii, Acinetobacter junii, Acinetobacter lwoffii, Actinomyces meyeri, Actinomyces pyogenes, Aeromonas caciae, Aeromonas hydrophila, Aeromonas schuhertii, Aeromonas veronii, Agrobacterium radiobacter, Alcaligenes faecalis, Anaerobiospirillum succiniciproducens, Anaerobiospirillum thomasii, Acranobacterium pyogenes, Barfonella bacilliformis, Bartonella henselae, Brucella abortus, Brucella melitensis, Calymmatobacterium granulomatis, Citrobacter amalonaticus, Citrobacter freundii, Coxiella burnetti, Edwardsiella tarda, Enterobacter aerogenes, Enterobacter cloacae, Enterobacter sakazakii, Enterobacter sp., Escherichia coli, Haemophilus aegypticus, Haemophilus aphrophilus, Haemophilus ducreyi, Haemophilus parahaemolyticus, Haemophilus parainfluenzae, Haemophilus paraphrophilus, Haemophilus segnis, Hafnia alvei, Klebsiella oxytoca, Klebsiella pneumoniae, Klebsiella rhinoscleromatis, Kluyvera ascorbata, Kluyvera cryocrescens, Legionella dumoffii, Legionella micdadei, Morganella morganii, Ochrobactrum anthropi, Pantoea agglomerans, Pasteurella gallinarum, Pasteurella pneumotropica, Plesiomonas shigelloides, Propionibacterium acnes, Proteus mirabilis, Proteus pennen, Proteus vulgaris, Pseudomonas putida, Rickettsia akari, Rickettsia australis, Rickettsia conorii, Salmonella choleraesius, Salmonella enterica, Salmonella paratyphi A, Salmonella paratyphi B, paratyphi C, Salmonella typhi, Salmonella typhinurium, Serratia ficaria, Senatia fonticola, Serratia grimesii, Serratia liquefaciens, Serratia marcescens, Serratia rudidaea, Shigella boydii, Shigella dysenteriae, Shigella flexneri, Shigella sonnei, Vibrio alginolyficus, Vibrio hollisae, Wigglesworfhia glossinidia, Xanthomonas campestris, Yersinia enterocolitica, Yersinia pestis, Yersinia pseudotuberculosis, Achromobacter piechaudii, Achromohacter xylosoxidans, Actinomadura madurae, Actinomadura pelletieri, Actinomyces bovis, Actinomyces naeslundii, Actinomyces viscosus, Afipia broomeae, Afipia felis, Bacteroides gracilis, Bilophila wadsworthia, Bordefella bronchiseptica, Bordetella parapertussis, Bordetella pertussis, Burkholderia cepacia, Burkholderia gladioli, Burkholderia mallei, Burkholderia pseudomallei, Campylobacter coli, Campylobacter fetus, Campylobacter jejuni, Campylobacter lari, Capnocytophaga canimorsus, Capnocytophaga cynodegmi, Capnocytophaga gingivalis, Capnocytophaga ochracea, Capnocytophaga sputigena, Chromobacterium violaceum, Comamonas terrigena, Comamonas testosteroni, Corynebacterium diphteriae, Corynebacterium minutissimum, Corynebacterium pseudotuberculosis, Corynebacterium urealyticum, Ehrlichia canis, Ehrlichia chaffeensis, Ehrlichia sennetsu, Eikenella corrodens, Eubacterium lentum, Francisella tularensis, Fusobacterium necrophorum, Fusobacterium nucleatum, Gardnerella vaginalis, Haemophilus influenzae, Helicobacter pylori, Kingella kingae, Methylobacterium extorquens, Methylobacterium mesophilicun, Mycobacterium africanum, Mycobacterium avium, Mycobacterium bovis, Mycobactrium chelonae, Mycobacterium intracellulare, Mycobacterium kanasasii, Mycobacterium leprae, Mycobacterium malmoense, Fusobacterium marinum, Mycobacterium scrofulaceum, Mycobacterium tuberculosis, Mycobacterium xenopi, Neisseria cinerea, Neisseria gonorrhoeae, Neisseria meningitidis, Nocardia asteroides, Nocardia nova, Nocardia octitidiscaviarum, Oligella urethralis, Oligella ureulytica, Peptoniphilus asaccharolyticus, Peptostreptococcus prevotii, Porohyromonas gingivalis, Prevotella buccae, Prevotella buccalis, Prevotella corporis, Prevotella denticola, Prevotella oralis, Rhodococcus equi, Rhodococcus erythropolis, Rhodococcus rhodochrous, Stenotrophomonas maltophilia, Streptobacillus moniliformis, Tropheryma whippley, Weeksella virosa, Actinobacillus actinomycetemcomitans, Actinobacillus equuli, Actinobacillus hominis, Actinobacillus suis, Actinobacillus ureae, Borrelia afzelii, Borrelia burgdorferi, Borrelia garninii, Borrelia hermsii, Borrelia hispanica, Chryseomonas luteola, Legionella dumoffii, Legionella micdadei, Legionella pneumophila, Moraxella (Branhamella) catarrhalis, Moraxella (Branhamella) nonliquefaciens, Moraxella (Branhamella) osloensis, Moraxella (Branhamella) phenylpyruvica, Pediococcus pentosaceus, Porphyromonas asaccharolytica, Providencia alcalifaciens, Providencia rettgeri, Providencia rustigianii, Providencia stuartii, Pseudomonas aeruginosa, Pseudomonas fluorescense, Psychrobacter immobilis, Vibrio cholerae, Vibrio parahaemolyticus, Vibrio vulnificus, Actinomyces israelii, Actinomyces odontolyticus, Arcanobacterium haemolyticum, Bacteroides eggerthii, Bacteroides fragilis, Bacteroides forsythus, Bacteroides merdae, Bacteroides putredinis, Chlamydiae trachomatis, Chlamydiae pneumoniae, Fusobacterium sulci, Leptospira biflexa, Leptospira interrogans, Mobiluncus mulieris, Mycoplasma pneumoniae, Peptostreptococcus anaerobicus, Peptostreptococcus magnus, Porphyromonas endodontalis, Rothia dentocariosa, Sphingobacterium multivorum, Sphingobacterium spiritovorum, Treponema pallidum et/ou Ureaplasma urealyticum sont détectées au moyen d'une analyse de courbes de fusion.

14. Procédé selon l'une quelconque des revendications 1 à 13, **caractérisé en ce que** la détermination des genres de bactéries au moyen d'une analyse de courbes de fusion s'effectue par dépassement d'une valeur de coupure prédéfinie par évaluation.

15. Kit de détection de bactéries, contenant au moins 10 bases consécutives des oligonucléotides SEQ ID:1 et SEQ ID NO:2, respectivement SEQ ID:3 et SEQ ID NO:4, ainsi que 10 bases consécutives des oligonucléotides SEQ ID NO:5, lequel est marqué à l'extrémité 3' à la fluorescéine, et au moins une séquence choisie parmi le groupe comprenant les SEQ ID NO:6 à SEQ ID NO:11 comme sondes d'hybridation, lesquelles sont parquées à l'extrémité 5' par un colorant fluorescent, et le cas échéant une polymérase d'ADN thermostable et des solutions de dNTP.
